(19) Europäisches Patentamt
European Patent Office
Office européen des brevets

(11) **EP 3 349 034 A2**

(12) **EUROPEAN PATENT APPLICATION**

(43) Date of publication:
**18.07.2018  Bulletin 2018/29**

(51) Int Cl.:
**G01S 7/52** *(2006.01)*   **G01S 15/89** *(2006.01)*

(21) Application number: **17205915.6**

(22) Date of filing: **07.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **13.01.2017   JP 2017004351**

(71) Applicant: **Konica Minolta, Inc.**
**Tokyo 100-7015 (JP)**

(72) Inventor: **Tsushima, Mineo**
**Tokyo, Tokyo 100-7015 (JP)**

(74) Representative: **Alton, Andrew**
**Urquhart-Dykes & Lord LLP**
**Arena Point**
**Merrion Way**
**Leeds LS2 8PA (GB)**

(54) **ULTRASOUND DIAGNOSTIC APPARATUS AND CONTROL METHOD THEREFOR**

(57)    To provide an ultrasonic diagnostic apparatus (100) that performs a synthetic aperture method using convergent transmission beamforming and is capable of changing configurations and process contents according to required calculation ability.

An ultrasonic diagnostic apparatus (100) including a first phasing adder (1041) that generates a first acoustic beam signal by performing phasing addition on the sequence of reception signals for a plurality of observation points in a first target region including at least a portion of the target region; a parameter calculator (1043) that calculates a parameter based on the first acoustic beam signal; a second phasing adder (1042) that generates a subframe acoustic beam signal by performing phasing addition on the sequence of reception signals based on the parameter for a plurality of observation points in a second target region which is all or a portion of the target region; a synthesizer (1044, 1045) that generates a frame acoustic beam signal by synthesizing the subframe acoustic beam signals; a controller (108) that determines whether to generate the ultrasonic image based on any one of the first acoustic beam signal and the frame acoustic beam signal; and an ultrasonic image generator (105) that generates the ultrasonic image.

*FIG. 1*

## Description

Background

Technological Field

The present disclosure relates to an ultrasonic diagnostic apparatus, and more particularly to a transmission and reception beamforming processing method, a control method, and an apparatus system configuration in an ultrasonic diagnostic apparatus.

Description of the Related art

An ultrasonic diagnostic apparatus transmits ultrasonic waves to the inside of a subject by a probe and receives reflected ultrasonic waves (echoes) caused by a difference in acoustic impedance between tissues of the subject. In addition, based on an electric signal obtained from the reception, an ultrasonic tomographic image indicating a structure of the internal tissue of the subject is generated and displayed. The ultrasonic diagnostic apparatus is widely used for morphological diagnosis of a living body because the ultrasonic diagnostic apparatus is less invasive to the subject and can observe a state of the internal tissues in real time with tomographic images and the like.

In the related art, as a reception beamforming method of a signal based on the received reflected ultrasonic wave, a method called a phasing addition method is generally used (for example, Masayasu Ito, Tsuyoshi Mochizuki "Ultrasonic Diagnostic Apparatus" published by Corona Publishing Co., Ltd., August 26, 2002 (P42-P45)). In this method, generally, when ultrasonic waves are transmitted to the subject, transmission beamforming is performed so that the ultrasonic beam is focused at a certain depth of the subject. In addition, observation points are set on or in the vicinity of the central axis of the transmitted ultrasonic beam. Therefore, the number of observation points is small in comparison with the area of the ultrasonic primary irradiation region, and thus, the utilization efficiency of ultrasonic waves is poor. In addition, when the observation point is at a position distant from the vicinity of the transmission focal point, there is also a problem in that the spatial resolution and the signal S/N ratio of the obtained acoustic beam signal are lowered. In addition, the ultrasonic primary irradiation region denotes a region through which the ultrasonic beam propagates.

Meanwhile, a reception beamforming method has been contrived to obtain a high-quality image with a high spatial resolution even in a region other than the vicinity of a transmission focal point by a synthetic aperture method (refer to, for example, "Virtual ultrasound sources in high resolution ultrasound imaging", S.I. Nikolov and J.A. Jensen, in Proc, SPIE - Progress in biomedical optics and imaging, vol. 3, 2002, P. 395-405). According to this method, by performing delay control taking into consideration both the propagation route of the ultrasonic transmission wave and the arrival time of the reflected wave at the transducer based on the propagation route of the ultrasonic transmission wave, it is possible to perform reception beamforming that also reflects reflected ultrasonic waves from the ultrasonic primary irradiation region located other than in the vicinity of the transmission focal point. As a result, it is possible to generate an acoustic beam signal (a signal based on the reflected ultrasonic wave from the observation point generated by reception beamforming) not only for the central axis of the transmitted ultrasonic beam but also for the entire ultrasonic primary irradiation region. In the synthetic aperture method, by virtually adjusting a transmission focus based on a plurality of reception signals for the same observation points obtained from a plurality of transmission events, an ultrasonic image having a high spatial resolution and a high S/N ratio can be obtained as compared with the reception beamforming method disclosed in Masayasu Ito, Tsuyoshi Mochizuki "Ultrasonic Diagnostic Apparatus" published by Corona Publishing Co., Ltd., August 26, 2002 (P42-P45).

Citation List

Patent Document

Patent Document 1: WO 2006/113445 A

Non-Patent Document

Non-Patent Document 1: Masayasu Ito, Tsuyoshi Mochizuki "Ultrasonic Diagnostic Apparatus" published by Corona Publishing Co., Ltd., August 26, 2002 (P42-P45)
Non-Patent Document 2: "Virtual ultrasound sources in high resolution ultrasound imaging", S.I. Nikolov and J.A. Jensen, in Proc, SPIE - Progress in biomedical optics and imaging, vol. 3, 2002, P. 395-405
Non-Patent Document 3: Synthetic Aperture Sequential Beamforming, Jacob Kortbek, et. al., IEEE Ultrasonics Symposium, 2-5 Nov. 2008 pp 966-969

Meanwhile, in the synthetic aperture method, from the viewpoint of improvement of ultrasonic wave utilization efficiency and resolution, it is preferable that the area of a region for generating an acoustic beam signal at one ultrasonic transmission event (hereinafter, referred to as "target region") is large, and it is more preferable that the entire ultrasonic primary irradiation region is set as the target region. However, as the area of the target region increases, the number of observation points (sites to be subjected to reception beamforming) existing in the target region increases in proportion to the area of the target region, so that the amount of calculation of the phasing addition considering the delay of transmission and reception increases. Therefore, if the area of the ultrasonic primary irradiation region increases, the calculation process of the phasing addition is performed at high velocity, so that hardware with a high calculation process ability is required. However, in a portable ultrasonic diagnostic apparatus, restrictions often arise with respect to the improvement of the calculation ability due to the size and arrangement of the apparatus, exhaust heat, a drivable time based on a battery, and the like. On the other hand, in the case of using ultrasonic diagnostic apparatuses with different calculation abilities according to the application, the number of ultrasonic diagnostic apparatuses is increased, which is inefficient.

Summary

The present invention has been made in view of the above problems, and an object of the present invention is to provide an ultrasonic diagnostic apparatus that performs a synthetic aperture method using convergent transmission beamforming and is capable of changing configurations and process contents according to required calculation ability.

To achieve the abovementioned object, according to an aspect of the present invention, an ultrasonic diagnostic apparatus reflecting one aspect of the present invention repeats several times a transmission event of transmitting a focused ultrasonic beam to a subject by using a probe having a plurality of transducers, generates a sequence of reception signals by receiving a reflected ultrasonic wave from the subject, and generates an ultrasonic image based on the sequence of reception signals, and the ultrasonic diagnostic apparatus comprises an ultrasonic signal processing circuit including: a transmitter that selects a transmission transducer column from the plurality of transducers for each transmission event while changing a focal point defining a converging position of the ultrasonic beam for each transmission event and transmits the ultrasonic beam from the transmission transducer column to a target region in the subject; a receiver that generates the sequence of reception signals for each transducer based on the reflected ultrasonic wave received from the target region by the probe; a first phasing adder that generates a first acoustic beam signal by performing phasing addition on the sequence of reception signals for a plurality of observation points in a first target region including a portion of the target region for each transmission event; a parameter calculator that calculates a parameter for generating a subframe acoustic beam signal based on the first acoustic beam signal; a second phasing adder that generates a subframe acoustic beam signal by performing phasing addition on the sequence of reception signals based on the parameter for a plurality of observation points in a second target region which is all or a portion of the target region; a synthesizer that generates a frame acoustic beam signal by synthesizing the subframe acoustic beam signals; a controller that determines whether to generate the ultrasonic image based on any one of the first acoustic beam signal and the frame acoustic beam signal; and an ultrasonic image generator that generates the ultrasonic image from any one of the first acoustic beam signal and the frame acoustic beam signal based on the determination of the controller.

Brief Description of the Drawings

The advantages and features provided by one or more embodiments of the invention will become more fully understood from the detailed description given hereinbelow and the appended drawings which are given by way of illustration only, and thus are not intended as a definition of the limits of the present invention:

Fig. 1 is a functional block diagram illustrating a configuration of an ultrasonic diagnostic system according to a first embodiment;
Fig. 2 is a schematic diagram illustrating a propagation route of an ultrasonic transmission wave by a transmission beamformer unit according to the first embodiment;
Fig. 3 is a functional block diagram illustrating a configuration of a reception beamformer unit according to the first embodiment;
Fig. 4 is a functional block diagram illustrating a configuration of a first phasing adder according to the first embodiment;
Fig. 5 is a schematic diagram illustrating a target region Bx, an observation line BL, and a representative point Qk according to the first embodiment;
Figs. 6A and 6B are schematic diagrams illustrating a propagation route of ultrasonic waves reaching a reception transducer Rm from a transmission aperture Tx through a representative point Qk according to the first embodiment;
Fig. 7 is a schematic diagram illustrating a relationship between delay times of reception transducers Rm;
Fig. 8 is a functional block diagram illustrating a configuration of a second phasing adder according to the first embodiment;
Fig. 9 is a schematic diagram illustrating a relationship between the position of an observation point Pij and a weight series calculated by a weight calculator according to the first embodiment;

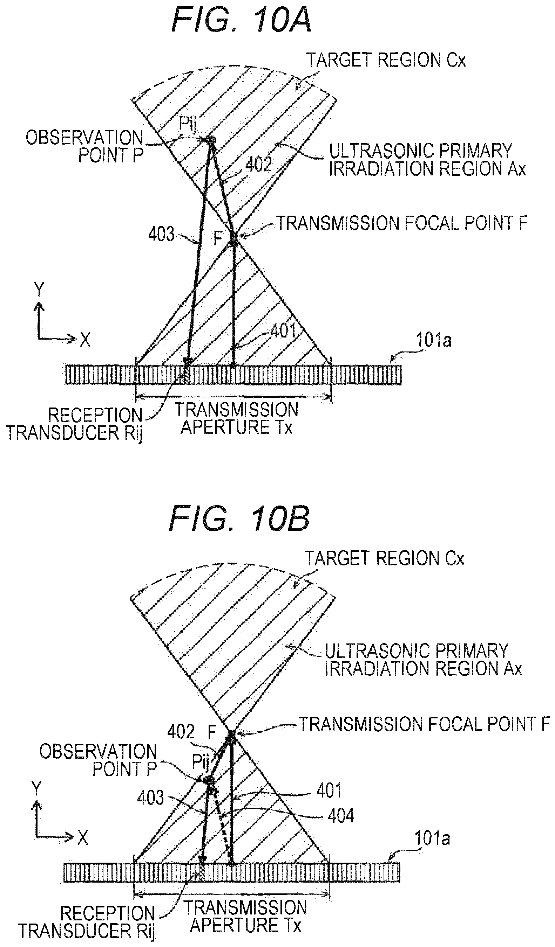

Figs. 10A and 10B are schematic diagrams illustrating a propagation route of ultrasonic waves reaching a reception transducer Rij from a transmission aperture Tx through an observation point Pij according to the first embodiment;
Figs. 11A and 11B are schematic diagrams illustrating a relationship between a delay amount and an ultrasonic velocity according to the embodiment;
Fig. 12A is a functional block diagram illustrating a configuration of a first synthesizer according to the first embodiment, and Fig. 12B is a functional block diagram illustrating a configuration of a second synthesizer according to the first embodiment;
Fig. 13 is a schematic diagram illustrating a subframe acoustic beam signal generation process in an acoustic beam signal development unit according to the first embodiment;
Fig. 14 is a schematic diagram illustrating a weighting synthesis process and a weight series in a weighting synthesizer according to the first embodiment;
Figs. 15A and 15B are schematic diagrams illustrating the maximum number of times of superimposition in an acoustic beam signal and the outline of an amplification process according to the first embodiment;
Fig. 16 is a flowchart illustrating a beamforming process of the reception beamformer unit in the case of using the second phasing adder according to the first embodiment;
Fig. 17 is a flowchart illustrating a beam-region acoustic beam signal generation operation according to the first embodiment;
Fig. 18 is a schematic diagram illustrating an acoustic beam signal generation operation with respect to a representative point Qk according to the first embodiment;
Fig. 19 is a flowchart illustrating a subframe acoustic beam signal generation operation according to the first embodiment;
Fig. 20 is a schematic diagram illustrating an acoustic beam signal generation operation on an observation point Pij according to the first embodiment;
Fig. 21 is a flowchart illustrating a beamforming process of the reception beamformer unit in the case of not using the second phasing adder according to the first embodiment;
Fig. 22 is a schematic diagram illustrating a relationship between the depth of an observation point and the S/N ratio of an acoustic beam signal when a gain correction value is changed according to a modified example;
Fig. 23 is a schematic diagram illustrating control for changing the width of a target region Cx according to the modified example;
Figs. 24A to 24C are schematic diagrams illustrating an ultrasonic diagnostic system, a portable main body, and a cart according to a second embodiment, respectively; and

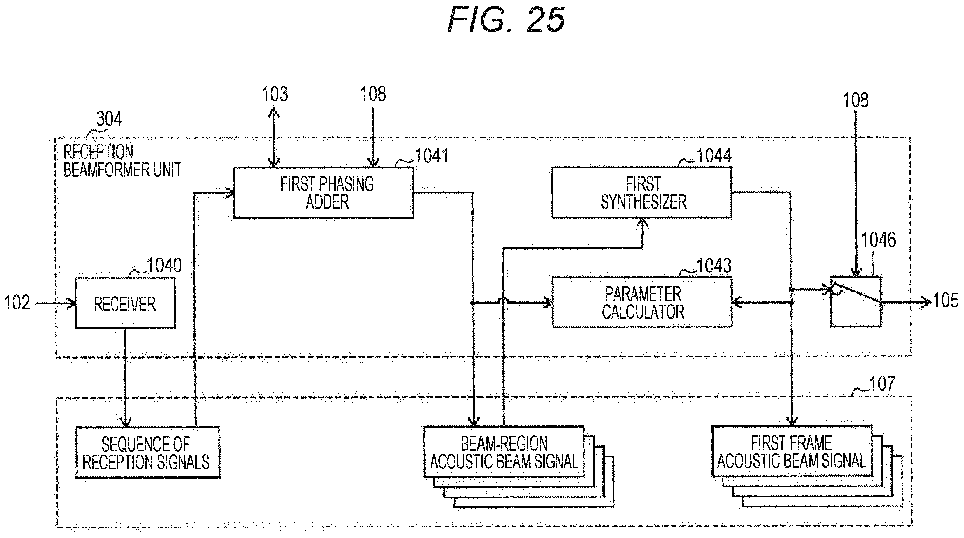

Fig. 25 is a functional block diagram illustrating a configuration of a reception beamformer unit according to the second embodiment.

Detailed Description of Embodiments

Hereinafter, one or more embodiments of the present invention will be described with reference to the drawings. However, the scope of the invention is not limited to the disclosed embodiments.

<First Embodiment>

<Overall Structure>

Hereinafter, an ultrasonic diagnostic apparatus 100 according to a first embodiment will be described with reference to the drawings.

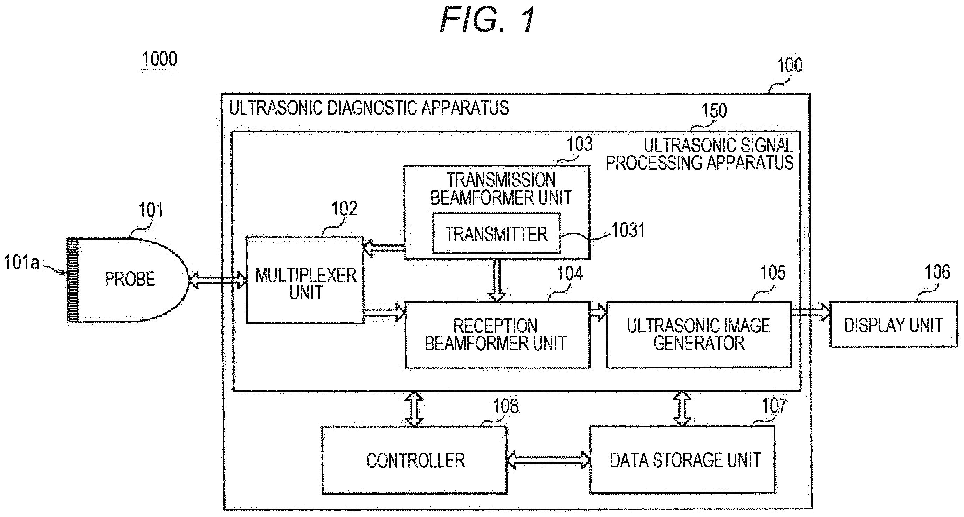

Fig. 1 is a functional block diagram of an ultrasonic diagnostic system 1000 according to the first embodiment. As illustrated in Fig. 1, the ultrasonic diagnostic system 1000 includes a probe 101 having a plurality of transducers 101a for transmitting ultrasonic waves toward a subject and receiving reflected waves of the ultrasonic waves, an ultrasonic diagnostic apparatus 100 for allowing the probe 101 to transmit and receive ultrasonic waves and generating an ultrasonic image based on an output signal from the probe 101, and a display unit 106 for displaying the ultrasonic image on a screen. Each of the probe 101 and the display unit 106 is connectable to the ultrasonic diagnostic apparatus 100. Fig. 1 illustrates a state in which the probe 101 and the display unit 106 are connected to the ultrasonic diagnostic apparatus 100. In addition, the probe 101 and the display unit 106 may be provided inside the ultrasonic diagnostic apparatus 100.

Configuration of Ultrasonic Diagnostic Apparatus 100>

The ultrasonic diagnostic apparatus 100 includes a multiplexer unit 102 for securing input and output for each of transducers used for transmission or reception among a plurality of transducers 101a of the probe 101, a transmission beamformer unit 103 for controlling the timing of application of a high voltage to the transducer 101a of the probe 101 in order to transmit ultrasonic waves, and a reception beamformer unit 104 for generating an acoustic beam signal by amplifying electric signals obtained by the plurality of transducers 101a based on the reflected wave of the ultrasonic wave received by the probe 101, performing A/D conversion, and reception beamforming. In addition, the ultrasonic diagnostic apparatus further includes an ultrasonic image generator 105 for generating an ultrasonic image (B-mode image) based on an output signal from the reception beamformer unit 104, a data storage unit 107 for storing an acoustic beam signal output by the reception beamformer unit 104 and an ultrasonic image output by the ultrasonic image generator 105, and a controller 108 for controlling each component.
Among the components, the multiplexer unit 102, the transmission beamformer unit 103, the reception beamformer unit 104, and the ultrasonic image generator 105 constitute an ultrasonic signal processing apparatus 150.
Each of the multiplexer unit 102, the transmission beamformer unit 103, the ultrasonic image generator 105, and the controller 108 constituting the ultrasonic diagnostic apparatus 100 is implemented with, for example, a hardware circuit such as a field programmable gate array (FPGA) or an application specific integrated circuit (ASIC). Alternatively, these components may be implemented by a programmable device such as a processor typified by a central processing unit (CPU) and software. The component can be formed as a single circuit part or can be formed as an assembly of a plurality of circuit parts. In addition, a plurality of components can be combined into a single circuit part or can be formed as an assembly of a plurality of circuit parts. In addition, the reception beamformer unit 104 will be described later.

The data storage unit 107 is a computer-readable recording medium, and for example, a flexible disk, a hard disk, an opto-magnetic disk, an optical disk, a semiconductor memory, or the like can be used. In addition, the data storage unit 107 may be a storage device externally connected to the ultrasonic diagnostic apparatus 100.

In addition, the ultrasonic diagnostic apparatus 100 according to the first embodiment is not limited to the ultrasonic diagnostic apparatus having the configuration illustrated in Fig. 1. For example, there may be no multiplexer unit 102, and the transmission beamformer unit 103 and the reception beamformer unit 104 may be directly connected to the transducers 101a of the probe 101. In addition, the transmission beamformer unit 103, the reception beamformer unit 104, a portion thereof, or the like may be incorporated into the probe 101. This is not limited to the ultrasonic diagnostic apparatus 100 according to this embodiment, and the same is applied to other ultrasonic diagnostic apparatuses according to other embodiments and modified examples described later.

<Configuration of Main Components of Ultrasonic Diagnostic Apparatus 100>

The ultrasonic diagnostic apparatus 100 according to the first embodiment includes the transmission beamformer unit 103 that allows the transducers 101a of the probe 101 to transmit ultrasonic beams and the reception beamformer unit 104 that generates an acoustic beam signal for generating an ultrasonic image by performing A/D conversion of an electric signal obtained by reception of a reflected ultrasonic wave in the probe 101. Therefore, in this specification, configurations and functions of the transmission beamformer unit 103 and the reception beamformer unit 104 will mainly be described. In addition, configurations other than those of the transmission beamformer unit 103 and the reception beamformer unit 104 can be the same as those used in well-known ultrasonic diagnostic apparatuses, and the beamformer units according to the embodiment can be used as substitutes for beamformer units of well-known ultrasonic diagnostic apparatuses.

Hereinafter, configurations of the transmission beamformer unit 103 and the reception beamformer unit 104 will be described.

1. Transmission Beamformer Unit 103

The transmission beamformer unit 103 is connected to the probe 101 through the multiplexer unit 102 and controls the timing of applying a high voltage to each of the plurality of transducers included in the transmission aperture Tx configured with the transmission transducer column corresponding to all or a portion of the plurality of transducers 101a existing in the probe 101 in order to transmit ultrasonic waves from the probe 101. The transmission beamformer unit 103 is configured with a transmitter 1031.

The transmitter 1031 performs a transmission process of transmitting a pulse-shaped transmission signal for transmitting the ultrasonic beam to each of the transducers included in the transmission aperture Tx among the plurality of transducers 101a existing in the probe 101 based on a transmission control signal from the controller 108. More specifically, the transmitter 1031 includes, for example, a clock generation circuit, a pulse generation circuit, and a delay circuit. The clock generation circuit is a circuit that generates a clock signal for determining the transmission timing of the ultrasonic beam. The pulse generation circuit is a circuit that generates a pulse signal for driving each transducer. The delay circuit is a circuit that sets the delay time of the ultrasonic beam transmission timing for each of the transducers and delays the transmission of the ultrasonic beam by the delay time to perform the focusing of the ultrasonic beam.

The transmitter 1031 repeatedly performs ultrasonic wave transmission while moving the transmission aperture Tx by the moving pitch Mp in the column direction at every ultrasonic wave transmission to perform the ultrasonic wave transmission from all the transducers 101a existing in the probe 101. In this embodiment, the moving pitch Mp is set to a pitch corresponding to one transducer, and the transmission aperture Tx moves by one transducer at every ultrasonic wave transmission. The moving pitch Mp is not limited to a pitch corresponding to one transducer, but the moving pitch Mp may be a pitch corresponding to, for example, 0.5 transducers. Information indicating the positions of the transducers included in the transmission aperture Tx is output to the data storage unit 107 through the controller 108. For example, when the total number of transducers 101a existing in the probe 101 is set to 192, the number of transducer columns constituting the transmission aperture Tx may be selected from, for example, 20 to 128, and the movement may be performed by one transducer at every ultrasonic wave transmission. Hereinafter, the ultrasonic wave transmission performed by the transmitter 1031 from the same transmission aperture Tx is referred to as a "transmission event".

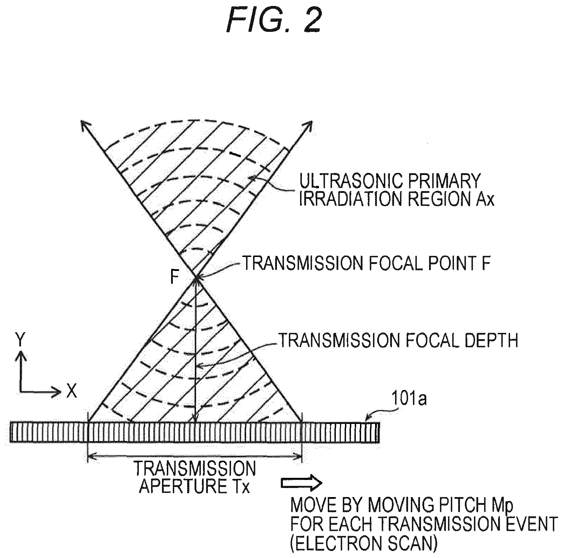

Fig. 2 is a schematic diagram illustrating the propagation route of the ultrasonic transmission wave by the transmission beamformer unit 103. In a certain transmission event, a column of transducers 101a (transmission transducer column) arrayed in an array shape that contributes to ultrasonic wave transmission is illustrated as the transmission aperture Tx. In addition, the column length of the transmission aperture Tx is called the transmission aperture length.

The transmission beamformer unit 103 controls the transmission timing of each of the transducers so that the transmission timing is more delayed for the transducer located closer to the center of the transmission aperture Tx. As a result, the ultrasonic transmission wave transmitted from the transducer column in the transmission aperture Tx is in a state where the wave front is focused (converges) at a certain point, that is, at a transmission focal point F (focal point) at a certain depth (focal depth) of the subject. The depth (focal depth) of the transmission focal point F can be arbitrarily set. Herein, the focal depth is the depth at which the ultrasonic transmission wave converges most in the direction (the x direction in Fig. 2) in which the transducers are aligned, that is, the depth in the y direction in which the width of the ultrasonic beam in the x direction is narrowest. The transmission focal point F is the center position in the x direction of the ultrasonic beam at the focal depth. However, the focal depth is fixed during a plurality of transmission events associated with one frame. That is, in a plurality of transmission events associated with one frame, the relative relationship between the transmission aperture Tx and the transmission focal point F does not change. The wave front focused at the transmission focal point F diffuses again, and the ultrasonic transmission wave propagates in an hourglass-shaped space delimited by two intersecting straight lines with the transmission aperture Tx as the base and the transmission focal point F as the node. That is, the ultrasonic wave radiated at the transmission aperture Tx gradually decreases the width on the space (horizontal axis direction in the figure) and minimizes the width at the transmission focal point F. The ultrasonic wave diffuses and propagates while increasing the width again as the ultrasonic wave progresses from a portion (the upper side in the figure) deeper than the transmission focal point F. This hourglass-shaped region is the ultrasonic primary irradiation region Ax. In addition, as described above, the ultrasonic primary irradiation region Ax may transmit the ultrasonic transmission wave so as to converge near one transmission focal point F.

2. Configuration of Reception Beamformer Unit 104

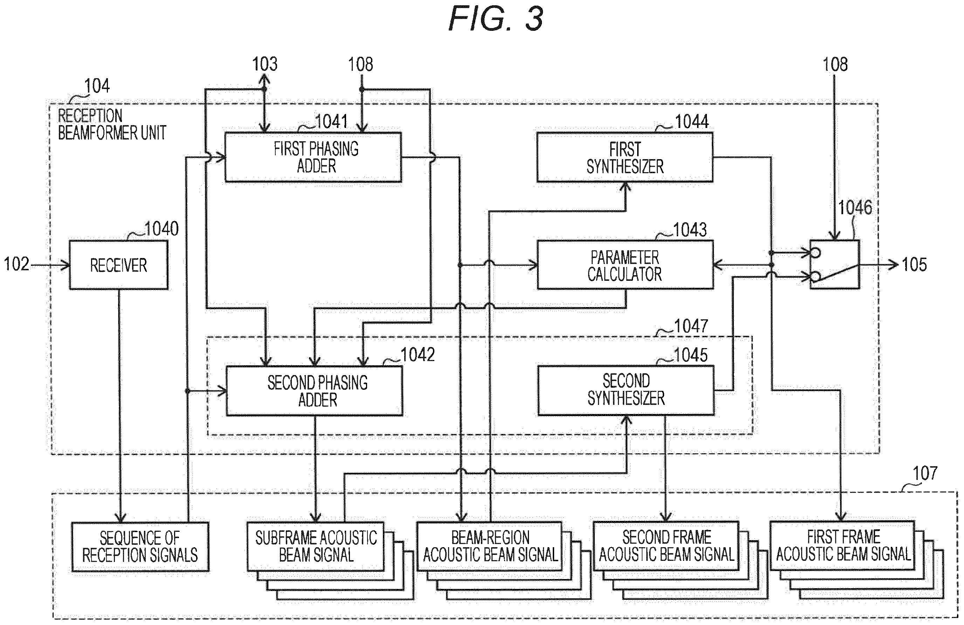

Based on the reflected wave of the ultrasonic wave received by the probe 101, the reception beamformer unit 104 generates an acoustic beam signal from an electric signal obtained by the plurality of transducers 101a. The "acoustic beam signal" is a signal after a phasing addition process has been performed on a certain observation point. The phasing addition process will be described later. Fig. 3 is a functional block diagram illustrating the configuration of the reception beamformer unit 104. As illustrated in Fig. 3, the reception beamformer unit 104 includes a receiver 1040, a first phasing adder 1041, a second phasing adder 1042, a parameter calculator 1043, a first synthesizer 1044, a second synthesizer 1045, and an output unit 1046.

Among the components, the second phasing adder 1042 and the second synthesizer 1045 constitute a high-performance arithmetic circuit 1047.

The high-performance arithmetic circuit 1047 is implemented by, for example, a programmable device such as a processor and software. As the processor, a CPU or a graphics processing unit (GPU) can be used, and the configuration using the GPU is called a general-purpose computing on graphics processing unit (GPGPU). In addition, the components of the reception beamformer unit 104 except the high-performance arithmetic circuit 1047, that is, the receiver 1040, the first phasing adder 1041, the parameter calculator 1043, the first synthesizer 1044, and the output unit 1046 are implemented by, for example, a hardware circuit such as a field programmable gate array (FPGA) and an application specific integrated circuit (ASIC), or a programmable device such as a processor and software. In addition, the second phasing adder 1042 and the second synthesizer 1045 have higher calculation ability than the first phasing adder 1041 and the first synthesizer 1044 respectively.

Hereinafter, the configuration of each component constituting the reception beamformer unit 104 will be described.

(1) Receiver 1040

The receiver 1040 is a circuit that is connected to the probe 101 through the multiplexer unit 102 and generates the reception signals (RF signals) by amplifying the electric signal obtained from the reception of the reflection ultrasonic wave by the probe 101 and then performing the A/D conversion in synchronization with the transmission event. The receiver generates the reception signals in a time-series manner in the order of the transmission events, outputs the reception signals to the data storage unit 107, and temporarily stores the reception signals in the data storage unit 107.

Herein, the reception signals (RF signals) are digital signals obtained by A/D converting electric signals converted from the reflected ultrasonic wave received by the transducers, and the reception signals constitute a sequence of signals in the transmission direction (the depth direction of the subject) of the ultrasonic waves received by the transducers.

In the transmission event, as described above, the transmitter 1031 allows the ultrasonic beams to be transmitted from each of the plurality of transducers included in the transmission aperture Tx among the plurality of transducers 101a existing in the probe 101. On the other hand, the receiver 1040 generates a sequence of reception signals for the transducers based on the reflected ultrasonic wave obtained by the transducers (hereinafter, referred to as "reception transducers") corresponding to some or all of the plurality of transducers 101a existing in the probe 101 in synchronization with the transmission events. It is preferable that the number of reception transducers is larger than the number of transducers included in the transmission aperture Tx. In addition, the number of reception transducers may be the total number of transducers 101a existing in the probe 101.

The transmitter 1031 repeats the ultrasonic wave transmission while moving the transmission aperture Tx by the moving pitch Mp in the column direction in synchronization with the transmission event and performs the ultrasonic wave transmission from the entire plurality of transducers 101a existing in the probe 101. The receiver 1040 generates a sequence of reception signals for each reception transducer in synchronization with the transmission event, and the generated reception signal is stored in the data storage unit 107.

(2) First Phasing Adder 1041

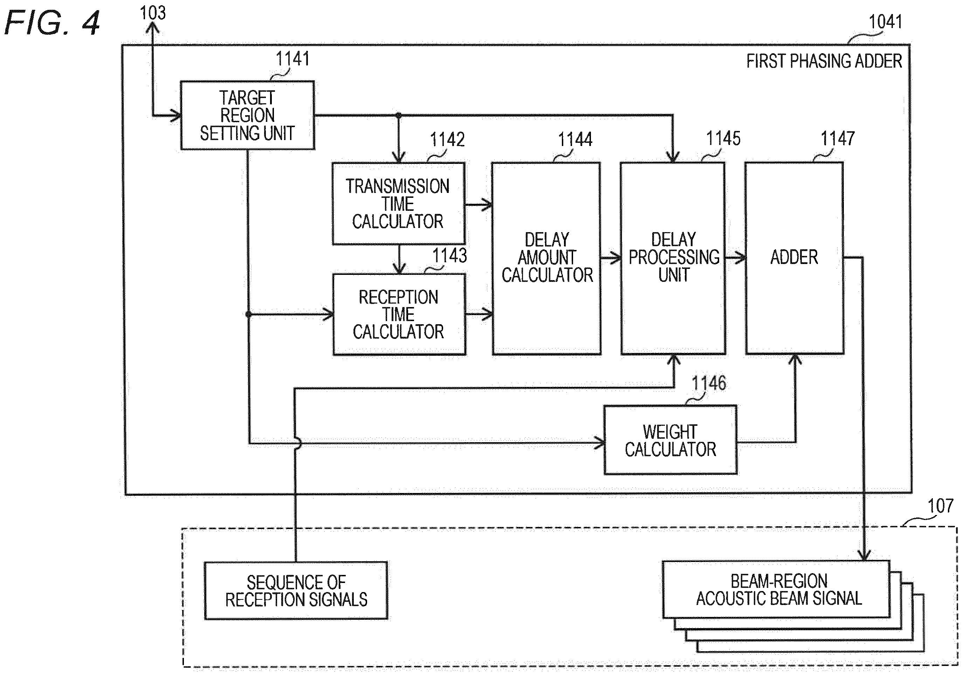

The first phasing adder 1041 sets a target region Bx for generating a subframe acoustic beam signal in the subject in synchronization with the transmission event. Next, an observation line BL passing through the transmission focal point F is set in the target region Bx. In this embodiment, the observation line BL is a straight line that passes through the transmission focal point F and the center line of the transmission aperture Tx and is perpendicular to the transducer column. In addition, the observation line BL may pass through the transmission focal point F and an arbitrary point in the transmission aperture Tx, but the present invention is not limited to the above case. Next, for each of the plurality of representative points Qk existing on the observation line BL, a sequence of reception signals received by each of the reception transducers Rm from the representative point Qk is subjected to phasing addition. The first phasing adder 1041 is a circuit that generates a beam-region acoustic beam signal by calculating a sequence of acoustic beam signals at each representative point Qk. Fig. 4 is a functional block diagram illustrating a configuration of the first phasing adder 1041. As illustrated in Fig. 4, the first phasing adder 1041 includes a target region setting unit 1141, a transmission time calculator 1142, a reception time calculator 1143, a delay amount calculator 1144, a delay processing unit 1145, a weight calculator 1146, and an adder 1147.

Hereinafter, the configuration of each component constituting the first phasing adder 1041 will be described.

i) Target Region Setting Unit 1141

The target region setting unit 1 141 sets a target region Bx for generating a subframe acoustic beam signal in the subject. The "target region" is a region on the signal where generation of a subframe acoustic beam signal is to be performed in the subject in synchronization with the transmission event. That is, the target region Bx is set as a set of observation target points where an acoustic beam signal is to be generated, for the convenience of calculation in synchronization with one transmission event. Herein, the "subframe acoustic beam signal" is a set of acoustic beam signals for all observation points existing in the target region Bx generated from one transmission event. The "subframe" denotes a unit obtained by one transmission event and forming collected signals corresponding to all observation points existing in the target region Bx, and a frame is obtained by synthesizing a plurality of subframes having different acquisition times.

The target region setting unit 1141 sets the target region Bx based on information indicating the position of the transmission aperture Tx acquired from the transmission beamformer unit 103 in synchronization with the transmission event.

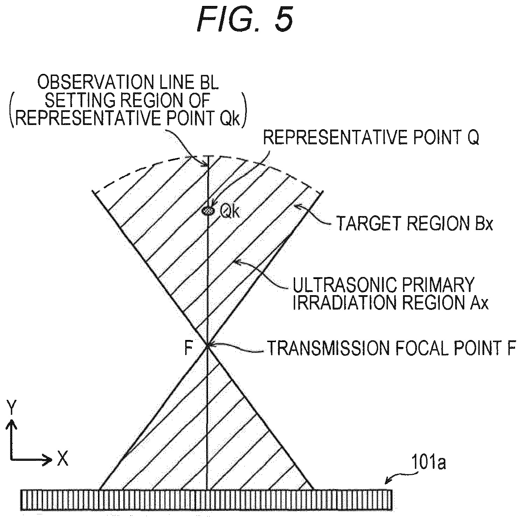

Fig. 5 is a schematic diagram illustrating the target region Bx. As illustrated in Fig. 5, the target region Bx is an arbitrary region existing in the ultrasonic primary irradiation region Ax, and in this embodiment, the target region Bx is the entire ultrasonic primary irradiation region Ax.

In addition, the target region setting unit 1141 sets the target line BL for generating the beam-region acoustic beam signal inside the target region Bx. In this embodiment, the target line BL is a straight line passing through the focal point F or the vicinity thereof. As described above, the target line BL should at least be a region on a straight line passing through the focal point F or the vicinity thereof and an arbitrary point on the transmission aperture Tx. Then, a beam-region acoustic beam signal is generated with respect to the representative point Qk set on the target line BL. In addition, as the reception aperture, the transmission aperture Tx is used as it is.

The set target region Bx is output to the controller 108, and the target line BL and the transmission aperture Tx acquired from the transmission beamformer unit 103 are output to the transmission time calculator 1142, the reception time calculator 1143, the delay processing unit 1145, and the weight calculator 1146.

ii) Transmission Time Calculator 1142

The transmission time calculator 1142 is a circuit that calculates the transmission time when the transmitted ultrasonic wave reaches the observation point P in the subject. In response to the transmission event, the transmission time calculator calculates the transmission time when the transmitted ultrasonic wave reaches the representative point Qk in the subject for an arbitrary representative point Qk existing on the target line BL based on the information indicating the position of the transducer included in the transmission aperture Tx and the information indicating the position of the target line BL acquired from the target region setting unit 1141.

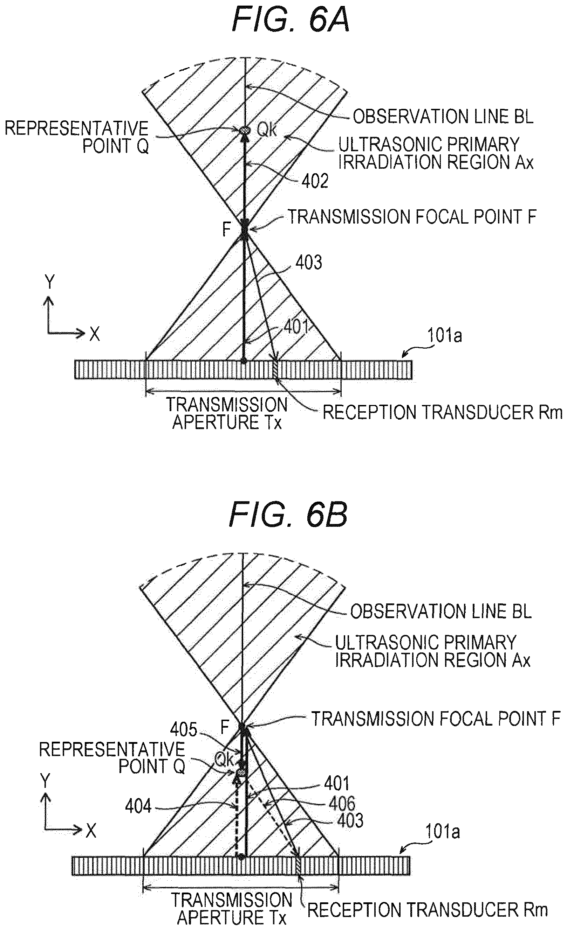

Figs. 6A and 6B are schematic diagrams illustrating a propagation route of ultrasonic waves that are emitted from the transmission aperture Tx, are reflected at the representative point Qk at an arbitrary position on the target line BL, and reach the reception transducer Rm located within the transmission aperture Tx. In addition, Fig. 6A illustrates the case where the depth of the representative point Qk is equal to or larger than the transmission focal depth, and Fig. 6B illustrates the case where the representative point Qk is shallower than the transmission focal depth.

The transmission wave radiated from the transmission aperture Tx passes through the route 401, and the wave front converges at the transmission focal point F and diffuses again. If the transmission wave reaches the representative point Qk while converging or diffusing and there is a change in the acoustic impedance at the representative point Qk, a reflected wave is generated, and the reflected wave returns to the reception transducers Rm in the transmission aperture Tx in the probe 101. Since the transmission focal point F is defined as a design value of the transmission beamformer unit 103, the length of the route 402 between the transmission focal point F and an arbitrary representative point Qk can be geometrically calculated.

A method of calculating the transmission time will be described more in detail below.

First, the case where the depth of the representative point Qk is equal to or larger than the transmission focal depth will be described with reference to Fig. 6A. In this case, the transmission time is calculated assuming that the transmission wave radiated from the transmission aperture Tx reaches the transmission focal point F through the route 401 and reaches the representative point Qk through the route 402 from the transmission focal point F. Therefore, the sum of the time of passing of the transmission wave through the route 401 and the time of passing of the transmission wave through the route 402 is the transmission time. More specifically, the transmission time is obtained by dividing the total route length, which is obtained by adding the length of the route 401 and the length of the route 402, by the propagation velocity of the ultrasonic wave in the subject.

On the other hand, the case where the representative point Qk is shallower than the transmission focal depth will be described with reference to Fig. 6B. In this case, the transmission time is calculated assuming that the time point at which the transmission wave radiated from the transmission aperture Tx reaches the transmission focal point F through the route 401 is the same as the time point at which the transmission wave reaches the transmission focal point F through the route 405 from the representative point Qk after the transmission wave reaches the representative point Qk through the route 404. That is, the value obtained by subtracting the time of passing of the transmission wave through the route 405 from the time of passing of the transmission wave through the route 401 is the transmission time. More specifically, the transmission time is obtained by dividing the route length difference, which is obtained by subtracting the length of the route 405 from the length of the route 401, by the propagation velocity of the ultrasonic wave in the subject.

The transmission time in the case where the representative point Qk is at the transmission focal depth may be calculated by using the same calculation method as the case where the representative point Qk is shallower than the transmission focal depth. This is because both of the length of the route 402 and the length of the route 405 become 0, and thus, in any calculation method, the transmission time coincides with the time of passing through the route 401.

The transmission time calculator 1142 calculates the transmission time when the transmitted ultrasonic wave reaches the observation point Qk in the subject for all the representative points Qk on the target line BL for one transmission event, and outputs the transmission time to the delay amount calculator 1144. In addition, the transmission time calculator 1142 outputs the length of the route 402 or the route 405 to the reception time calculator 1143 for all the representative points Qk on the target line BL for one transmission event.

iii) Reception Time Calculator 1143

The reception time calculator 1143 is a circuit that calculates the reception time when the reflected wave from the representative point Q reaches each of the reception transducers Rm included in the transmission aperture Tx. In response to the transmission event, the reception time calculator calculates the reception time when the transmitted ultrasonic wave is reflected at the representative point Qk in the subject and reaches each of the reception transducers Rm of the transmission aperture Tx for any arbitrary representative point Qk existing on the target line BL based on the information indicating the position of the reception transducer Rk acquired from the target region setting unit 1141 and the information indicating the position of the target line BL.

As described above, the transmission wave reaching the representative point Qk generates a reflected wave if there is a change in the acoustic impedance at the representative point Qk, and the reflected wave returns to each of the reception transducers Rm in the transmission aperture Tx in the probe 101. At this time, similarly to the transmission ultrasonic beam, the reception time calculator 1143 calculates a route from the representative point Qk to the reception transducer Rm with the transmission focal point F as a reference.

First, the concept of a method of calculating the reception time will be described with reference to Figs. 6A and 6B. Note that the calculation can be simplified as described later.

First, the case where the depth of the representative point Qk is equal to or larger than the transmission focal depth will be described with reference to Fig. 6A.

In this case, the reception time is calculated assuming that the reflected wave reflected by the representative point Qk reaches the transmission focal point F through the route 402 and reaches the reception transducer Rm through the route 403 from the transmission focal point F. Therefore, the sum of the time of passing through the route 402 and the time of passing through the route 403 is the reception time.

On the other hand, the case where the representative point Qk is shallower than the transmission focal depth will be described with reference to Fig. 6B. In this case, the reception time is calculated assuming that the time point at which the reflected wave reflected by the transmission focal point F reaches the reception transducer Rm through the route 406 after reaching the representative point Qk through the route 405 is the same as the time point at which the reflected wave directly reaches the reception transducer Rm through the route 403. In other words, the time for the reflected wave reflected by the representative point Qk to reach the reception transducer Rm is the time obtained by subtracting only the time necessary for passing through the route 405 from the time for the reflected wave reflected by the transmission focal point F to reach the reception transducer Rm through the route 403. Therefore, the value obtained by subtracting the time of passing through the route 405 from the time of passing through the route 403 is the reception time.

Herein, the length of the route 402 or the route 405 for each representative point Qk is the same as the length of the route 402 or the route 405 for each representative point Qk which the transmission time calculator 1142 calculates as a portion of the transmission time. Therefore, in this embodiment, the length of the route 402 or the route 405 for each representative point Qk calculated by the transmission time calculator 1142 is used for calculating the reception time. In addition, the length of the route 403 depends only on the positional relationship between the transmission focal point F and the reception transducer Rm. In other words, the difference in the reception time between the two reception transducers $Rm_1$ and $Rm_2$ with respect to the same representative point Q does not depend on the position of the representative point at all. That is, the difference in the reception time between the two reception transducers $Rm_1$ and $Rm_2$ with respect to the same representative point Q is constant with respect to the representative point $Qk_1$, the representative point $Qk_2$, and the representative point $Qk_3$.

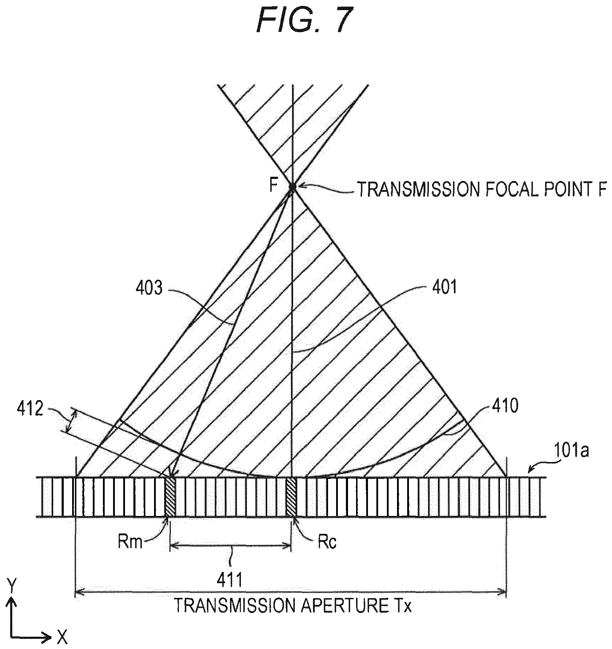

Hereinafter, a more detailed description will be given with reference to Fig. 7. The length of the route 403 is determined by the positional relationship between the reception transducer Rm and the transmission focal point F. The difference between the reception time of the reception transducer Rm and the reception time of the reception transducer Rc located at the center of the transmission aperture Tx is the time required for the ultrasonic wave to propagate through a distance 412 between the circular arc 410 that is in contact with the reception transducer Rc with the transmission focal point F as the center and the reception transducer Rm.

Therefore, the reception time calculator 1143 calculates the reception time for each representative point Qk with respect to the reception transducer Rc by using the length of the route 401 corresponding to the length of the route 403 of the reception transducer Rc and the length of the route 402 or the route 405 for each representative point Qk calculated by the transmission time calculator 1142. In addition, the difference in the reception time between the reception transducer Rc and each of the reception transducers Rm is calculated by dividing the distance 412 for each of the reception transducers Rm by the propagation velocity of the ultrasonic wave. Then, the reception time for each representative point Qk with respect to the reception transducer Rc and the difference in the reception time between each of the reception transducers Rm and the reception transducer Rc are output to the delay amount calculator 1144.

iv) Delay Amount Calculator 1144

The delay amount calculator 1144 is a circuit that calculates the total propagation time to each of the reception transducers Rm in the transmission aperture Tx based on the transmission time and the reception time and calculates the delay amount to be applied to the sequence of the reception signals for each of the reception transducers Rm based on the total propagation time. The delay amount calculator 1144 acquires the transmission time when the transmitted ultrasonic wave reaches the representative point Qk, the reception time at which the ultrasonic wave reflected at the representative point Qk reaches the reception transducer Rc, and the difference in the reception time between the reception transducer Rc and each of the reception transducers Rm. Then, the delay amount calculator calculates the total propagation time until the transmitted ultrasonic wave reaches each of the reception transducers Rm and calculates the delay amount for each of the reception transducers Rm based on a difference in the total propagation time for each of the reception transducers Rm. The total propagation time for the reception transducer Rc for each representative point Qk can be obtained as a sum of the transmission time for the representative point Qk and the reception time for the reception transducer Rc. In addition, the total propagation time for each of the reception transducers Rm can be obtained by adding the difference in the reception time between the reception transducer Rc and each of the reception transducers Rm to the total propagation time of the reception transducer Rc with respect to the same representative point Qk. The delay amount calculator 1144 calculates the delay amount to be applied to a sequence of the reception signals for the reception transducer Rc with respect to all the representative points Qk existing on the target line BL and outputs the delay amount together with the difference in the reception time between the reception transducer Rc and each of the reception transducers Rm to the delay processing unit 1145.

### v) Delay Processing Unit 1145

The delay processing unit 1145 is a circuit that identifies the reception signal corresponding to the delay amount for each of the reception transducers Rm from a sequence of reception signals for the reception transducer Rm in the transmission aperture Tx as a reception signal corresponding to the reception transducer Rm based on the reflection ultrasonic wave from the representative point Qk.

In response to the transmission event, the delay processing unit 1145 receives, as an input, information indicating the position of the reception transducer Rm and the position of the target line BL from the target region setting unit 1141, the reception signal corresponding to the reception transducer Rm from the data storage unit 107, and the delay amount to be applied to a sequence of the reception signals for each of the reception transducers Rm from the delay amount calculator 1144. Then, the delay processing unit identifies the reception signal corresponding to the time obtained by subtracting the delay amount for each of the reception transducers Rm from a sequence of reception signals corresponding to each of the reception transducers Rm as a reception signal based on the reflected wave from the representative point Qk and outputs the identified reception signal to the adder 1147.

More specifically, the delay processing unit 1145 performs a delay process on a sequence of reception signals for each of the reception transducers Rm so as to cancel the difference in the reception time between the reception transducer Rc and each of the reception transducers Rm. The delay processing unit can extract a set of the reception signals based on the reflected ultrasonic waves from the same representative point Qk by extracting the reception signals corresponding to the same time from a sequence of the reception signals after the delay process.

### vi) Weight Calculator 1146

The weight calculator 1146 is a circuit that calculates a weight series (reception apodization) for each of the reception transducers Rm.

The weight series is a sequence of weight coefficients applied to the reception signal corresponding to each of the transducers in the transmission aperture Tx. The weight series has a symmetric distribution with respect to the transmission focal point F as a center. The weight series is set such that the weight for the transducer located at the center of the transmission aperture Tx in the column direction is maximized, and the central axis of the weight distribution coincides with the transmission aperture central axis. The shape of the weight series is, for example, a Hamming window, a Hanning window, or a rectangular window.

The weight calculator 1146 calculates a weight series for each of the reception transducers Rm based on input information indicating the position of the transmission aperture Tx output from the target region setting unit 1141 and outputs the calculated weight series for each representative point Qk to the adder 1147.

### vii) Adder 1147

The adder 1147 receives, as an input, the reception signal identified corresponding to each of the reception transducers Rm output from the delay processing unit 1145 and the weight profile output from the weight calculator 1146 and generates an acoustic beam signal for the representative point Qk by multiplying and adding the weight of each of the reception transducers Rm to the reception signal identified corresponding to each of the reception transducers Rm. The delay processing unit 1145 arranges the phases of the reception signals detected by the reception transducers Rm located in the transmission aperture Tx, and the adder 1147 performs an addition process, so that it is possible to increase the signal S/N ratio by superimposing the reception signals received by the reception transducers Rm based on the reflected wave from the representative point Qk and to extract the reception signals from the representative point Qk.

The above-described processes are summarized as follows. A sequence of reception signals for the reception transducer Rm is denoted by Rf(m, t). Herein, m is an identifier indicating the reception transducer, and t is the time point at which the reception transducer Rc receives the reflected ultrasonic wave from the representative point Qk. In addition, the weighting factor for the reception transducer Rm is denoted by A(m). In addition, the difference in the reception time between the reception transducer Rm and the reception transducer Rc is denoted by d(m). At this time, the acoustic beam signal Das (k) for the representative point Qk is given by the following mathematical formula.

[Mathematical Formula 1]

$$Das(k) = \sum_m \left\{ A(m) \times Rf\left(m, t + d(m)\right) \right\}$$

Through the above-described processes, it is possible to generate the acoustic beam signals for all the representative points Qk on the target line BL associated with one transmission event. The generated beam-region acoustic beam signal is output to the data storage unit 107 for storage.

### (3) Second Phasing Adder 1042

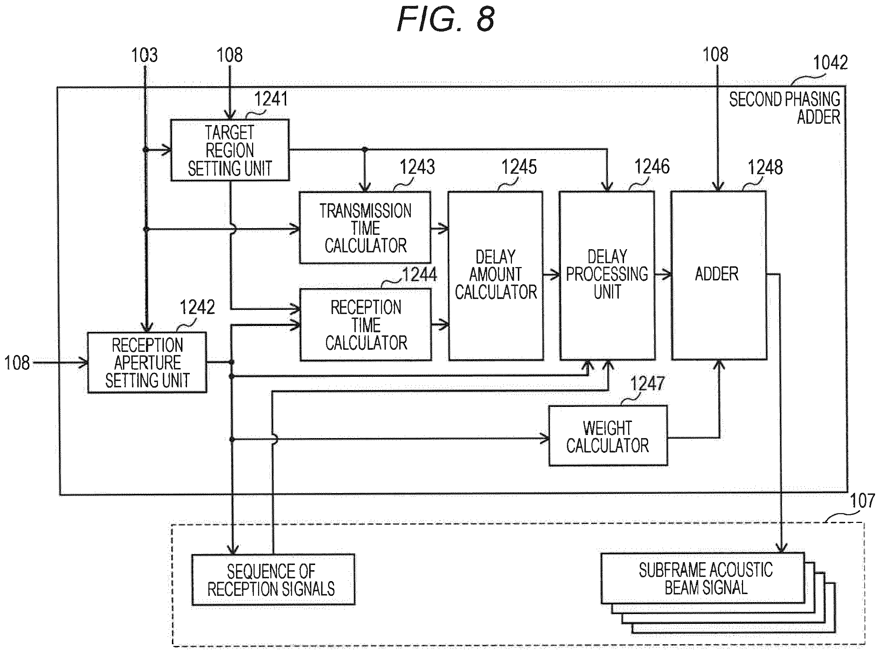

The second phasing adder 1042 sets a target region Cx for generating a subframe acoustic beam signal in the subject in synchronization with the transmission event. Next, for each of the plurality of observation points Pij existing in the target region Cx, a sequence of reception signals received by each of the reception transducers Rk from the observation point is subjected to phasing addition. Then, the second phasing adder 1042 is a circuit that generates a subframe acoustic beam signal by calculating a sequence of acoustic beam signals at each observation point. Fig. 8 is a functional block diagram illustrating a configuration of the second phasing adder 1042. As illustrated in Fig. 8, the second phasing adder 1042 includes a target region setting unit 1241, a reception aperture setting unit 1242, a transmission time calculator 1243, a reception time calculator 1244, a delay amount calculator 1245, a delay processing unit 1246, a weight calculator 1247, and an adder 1248.

Hereinafter, the configuration of each component constituting the second phasing adder 1042 will be described. In addition, detailed description of configurations having almost the same functions as the configurations having the same names constituting the first phasing adder 1041 will be omitted.

i) Target Region Setting Unit 1241

The target region setting unit 1241 sets a target region Cx for generating a subframe acoustic beam signal in the subject.

The target region setting unit 1241 sets the target region Cx based on the target region Bx set by the target region setting unit 1241 of the first phasing adder 1041 in synchronization with the transmission event. In this embodiment, the target region Cx is the entire target region Bx.

The set target region Cx is output to the transmission time calculator 1243, the reception time calculator 1244, and the delay processing unit 1246.

ii) Reception Aperture Setting Unit 1242

The reception aperture setting unit 1242 is a circuit that selects a transducer column (reception transducer column) which corresponds to a portion of a plurality of transducers existing in the probe 101 and of which the center coincides with the transducer that is spatially closest to the observation point as the reception transducer based on the control signal from the controller 108 and the information indicating the position of the transmission aperture Tx from the transmission beamformer unit 103 and sets the reception aperture Rx.

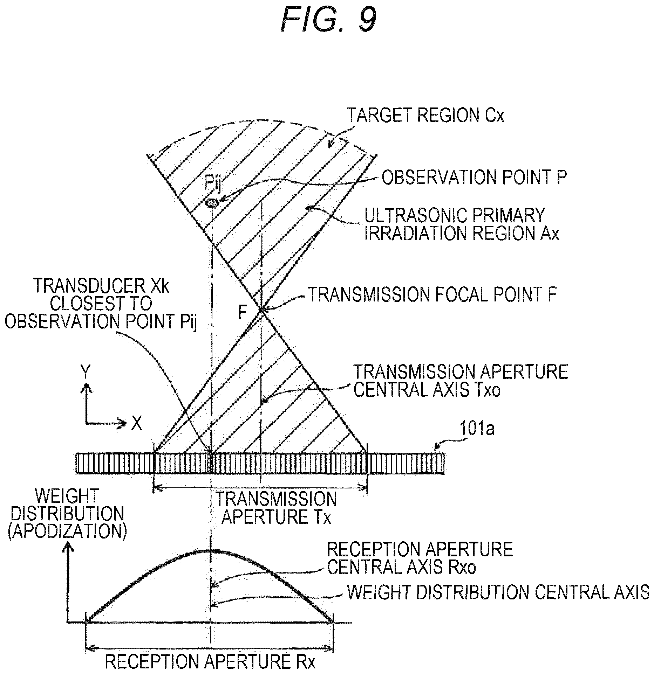

Fig. 9 is a schematic diagram illustrating the relationship between the reception aperture Rx and the transmission aperture Tx set by the reception aperture setting unit 1242. As illustrated in Fig. 9, the reception aperture Rx is set so that the center thereof coincides with the transducer Xk that is spatially closest to the observation point Pij. In addition, the position of the reception aperture Rx is determined for each observation point Pij and does not change based on the position of the transmission aperture Tx that changes in synchronization with the transmission event. That is, even in different transmission events, in the process of generating the acoustic beam signals for the observation points Pij located at the same position, the phasing addition is performed based on the reception signals acquired by the reception transducers Rk in the same reception aperture Rx.

In addition, in order to receive the reflected waves from the entire ultrasonic primary irradiation region Ax, it is preferable that the number of transducers included in the reception aperture Rx is set to be equal to or larger than the number of transducers included in the transmission aperture Tx in the corresponding transmission event. The number of transducer columns constituting the reception aperture Rx may be, for example, 32, 64, 96, 128, 192, or the like.

The setting of the reception aperture Rx is performed at least as many times as the maximum number of observation points Pij in the column direction. In addition, the setting of the reception aperture Rx may be performed gradually in synchronization with the transmission event. Alternatively, after the completion of all the transmission events, the setting of the reception aperture Rx corresponding to each transmission event may be performed collectively for the number of times of the transmission events.

The information indicating the position of the selected reception aperture Rx is output to the data storage unit 107 through the controller 108.

The data storage unit 107 outputs the information indicating the position of the reception aperture Rx and the reception signal corresponding to the reception transducer to the transmission time calculator 1243, the reception time calculator 1244, the delay processing unit 1246, and the weight calculator 1247.

iii) Transmission Time Calculator 1243

The transmission time calculator 1243 is a circuit that calculates the transmission time when the transmitted ultrasonic wave reaches the observation point Pij in the subject. In response to the transmission event, the transmission time calculator calculates the transmission time when the transmitted ultrasonic wave reaches the observation point Cij in the subject for each observation point Pij existing in the region Cx based on the information indicating the position of the transducer included in the transmission aperture Tx acquired from the data storage unit 107 and the information indicating the position of the target region Cx acquired from the target region setting unit 1241. Since the transmission time calculation method is the same as the transmission time calculator 1142, the description thereof will be omitted herein.

The transmission time calculator 1243 calculates the transmission time when the transmitted ultrasonic wave reaches the observation point Pij in the subject with respect to all the observation points Pij in the target region Cx for one transmission event and outputs the transmission time to the delay amount calculator 1245.

iv) Reception Time Calculator 1244

The reception time calculator 1244 is a circuit that calculates the reception time when the reflected wave from the observation point Pij reaches each of the reception transducers Rk included in the reception aperture Rx. In response to the transmission event, the reception time calculator calculates the reception time when the transmitted ultrasonic wave is reflected by the observation point Pij in the subject and reaches each of the reception transducers Rk of the reception aperture Rx for an arbitrary observation point Pij existing in the target region Cx based on the information indicating the position of the reception transducer Rk acquired from the data storage unit 107 and the information indicating the position of the target region Cx acquired from the target region setting unit 1141.

Unlike the reception time calculator 1143, the reception time calculator 1244 calculates the reception time assuming that the reflected wave passes through the shortest route 403 from the observation point Pij to the reception transducer Rk.

The reception time calculator 1244 calculates the reception time when the transmitted ultrasonic wave is reflected at the observation point Pij for all the observation points Pij existing in the target region Cx for one transmission event and reaches each of the reception transducers Rk and outputs the reception time to the delay amount calculator 1245.

v) Delay Amount Calculator 1245

The delay amount calculator 1245 is a circuit that calculates the total propagation time to each of the reception transducers Rk in the reception aperture Rx based on the transmission time and the reception time and calculates the delay amount to be applied to the sequence of the reception signals for each of the reception transducers Rk based on the total propagation time. The delay amount calculator 1245 acquires the transmission time when the transmitted ultrasonic wave reaches the observation point Pij and the reception time when the ultrasonic wave is reflected at the observation point Pij and reaches each of the reception transducers Rk. Then, the delay amount calculator calculates the total propagation time until the transmitted ultrasonic wave reaches each of the reception transducers Rk and calculates the delay amount for each of the reception transducers Rk based on the difference in the total propagation time for each of the reception transducers Rk. The delay amount calculator 1245 calculates the delay amount to be applied to a sequence of the reception signals for each of the reception transducers Rk with respect to all the observation points Pij existing in the target region Cx and outputs the delay amount to the delay processing unit 1246.

vi) Delay Processing Unit 1246

The delay processing unit 1246 is a circuit that identifies the reception signal corresponding to the delay amount for each of the reception transducers Rk from a sequence of reception signals for the reception transducer Rk in the reception aperture Rx as the reception signal corresponding to the reception transducer Rk based on the reflected ultrasonic wave from the observation point Pij.

In response to the transmission event, the delay processing unit 1246 receives, as an input, the information indicating the position of the reception transducer Rk from the reception aperture setting unit 1242, the reception signal corresponding to the reception transducer Rk from the data storage unit 107, the Information indicating the position of the acquired target region Cx acquired from the target region setting unit 1241, and the delay amount to be applied to a sequence of the reception signals for each of the reception transducers Rk from the delay amount calculator 1245. Then, the delay processing unit identifies the reception signal corresponding to the time obtained by subtracting the delay amount for each of the reception transducers Rk from a sequence of reception signals corresponding to each of the reception transducers Rk as the reception signal based on the reflected wave from the observation point Pij and outputs the identified reception signal to the adder 1248.

vii) Weight Calculator 1247

The weight calculator 1247 is a circuit that calculates the weight series for each of the reception transducers Rk so that the weight for the transducer located at the center in the column direction of the reception aperture Rx is maximized.

As illustrated in Fig. 9, the weight series is a sequence of weight coefficients applied to the reception signals corresponding to the reception transducers in the reception aperture Rx. The weight series has a symmetric distribution centered on the transmission focal point F. As the shape of the distribution of the weight series, a Hamming window, a Hanning window, a rectangular window, or the like can be used, and the shape of the distribution is not particularly limited. The weight series is set so that the weight for the transducer located at the center of the reception aperture Rx in the column direction is maximized, and the central axis of the weight distribution coincides with the reception aperture central axis Rxo. The weight calculator 1247 receives, as an input, information indicating the position of the reception transducer Rk output from the reception aperture setting unit 1242, calculates a weight series for each of the reception transducers Rk, and outputs the calculation result to the adder 1248.

viii) Adder 1248

The adder 1248 is a circuit that receives, as inputs, the reception signals identified corresponding to the reception transducers Rk output from the delay processing unit 1246, adds the reception signals, and generates the phasing-added acoustic beam signals for the observation point Pij. Alternatively, the adder is configured to receive, as an input, the weight series for each of the reception transducers Rk output from the weight calculator 1247 and to generate an acoustic beam signal for the observation point Pij by multiplying and adding the weight for each of the reception transducers Rk to the reception signal identified corresponding to each of the reception transducers Rk.

The adder 1248 generates acoustic beam signals of subframes for all the observation points Pij existing in the target region Cx in synchronization with the transmission event. The generated acoustic beam signals of the subframes are output to the data storage unit 107 for storage.

(4) Parameter Calculator 1043

The parameter calculator 1043 is a circuit that allows the second phasing adder 1042 to calculate a parameter for performing the reception beamforming based on the beam-region acoustic beam signal generated by the first phasing adder 1041 and/or the first frame acoustic beam signal generated by the first synthesizer 1044 to be described later. Herein, the parameter is, for example, a propagation velocity (hereinafter, referred to as an "ultrasonic velocity") of the ultrasonic wave in the subject, a weight (hereinafter, referred to as a "gain correction value") to the depth of the observation point, information specifying the size of the target region Cx, and the like.

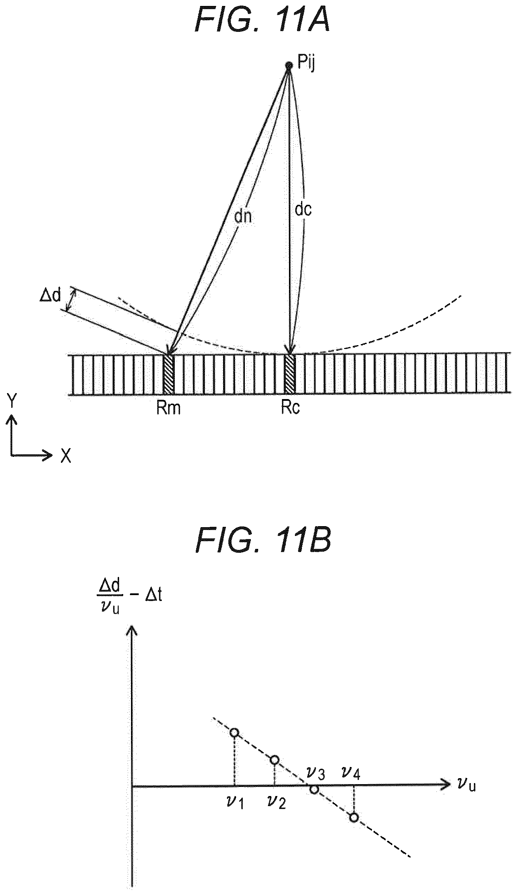

In this embodiment, the ultrasonic velocity in the subject is calculated as a parameter. The ultrasonic velocity in the subject is used for coordinate transformation (time difference is transformed into a difference in depth) when converting the acoustic beam signal to a B-mode image and for calculation of the delay amount in the phasing addition as described above. As illustrated in the schematic diagram of Fig. 11A, when generating the acoustic beam signal based on the reflected wave from the observation point Pij, the delay amount is set so that the timing of the reception signal based on the reflected wave from the observation point Pij in the transducer Rc and the timing of the reception signal based on the reflected wave from the observation point Pij in the transducer Rm are the same. At this time, the delay amount is calculated by using the geometrical distance dc between the transducer Rc and the observation point Pij, the geometrical distance dm between the transducer Rm and the observation point Pij, and the ultrasonic velocity vu. More specifically, the phasing addition is performed on the premise that the timing with respect to the reception signal based on the reflected wave from the observation point Pij in the transducer Rm is delayed by $\Delta d/vu$ from the timing with respect to the reception signal based on the reflected wave from the observation point Pij in the transducer Rc by using $\Delta d = dm - dc$.

At this time, as the ultrasonic velocity vu, a value estimated from the characteristics of the subject is used. However, the ultrasonic velocity depends on the hardness of the tissue in the subject, and the estimated value may contain an error. Herein, in the case where the error of the estimated value vu of the ultrasonic velocity is large, that is, in the case where the difference between the real time difference $\Delta t$ between the time point at which the reflected wave from the observation point Pij reaches the transducer Rc and the time point at which the reflected wave reaches the transducer Rm and the calculational delay amount difference $\Delta d/vu$ is large, the reception focus of the phasing addition is not matched (deviated). Fig. 11B is a schematic diagram illustrating the relationship between the calculational ultrasonic velocity vu and the deviation $\Delta d/vu - \Delta t$ between the delay amount and the true time difference. However, it is difficult to accurately estimate the ultrasonic velocity in advance.

Therefore, the parameter calculator 1043 allows the first phasing adder 1041 to generate a plurality of beam-region acoustic beam signals having different calculational ultrasonic velocities vu only for the same target line BL and performs estimation of the ultrasonic velocity vu. More specifically, for example, the parameter calculator 1043 compares the beam-region acoustic beam signals corresponding to the ultrasonic velocities v1, v2, v3, and v4 generated for the same target line BL and outputs the ultrasonic velocity corresponding to the beam-region acoustic beam signal having the largest turbulence to the second phasing adder 1042 as the ultrasonic velocity. This is because, as the deviation between the calculational ultrasonic velocity and the actual ultrasonic velocity becomes small, the reception focus of the phasing addition is matched, that is, the S/N ratio of the acoustic beam signal increases. That is, this is because, as the deviation between the calculational ultrasonic velocity and the actual ultrasonic velocity becomes small, the time difference of the peak timing in the plurality of reception signals based on the reflected ultrasonic waves from one ultrasonic reflection source becomes small. Thus, the peak becomes sharp, that is, the peak signal value becomes large and the full width at half height of time becomes short, and an acoustic beam signal with high distance resolution (time resolution) can be obtained.

The parameter calculator 1043 outputs the calculated parameter to the second phasing adder 1042.

(5) First Synthesizer 1044

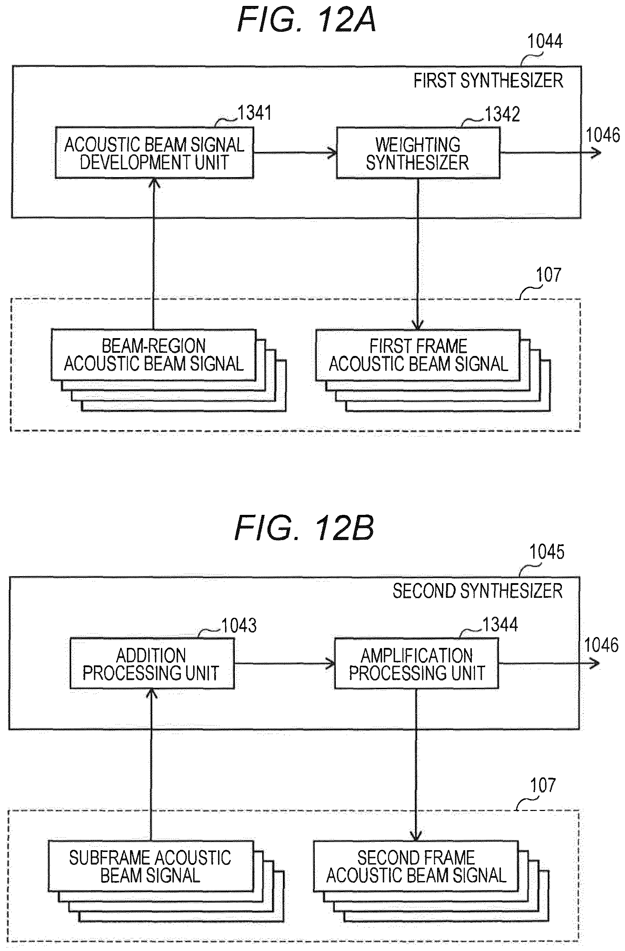

The first synthesizer 1044 is a circuit that generates the subframe acoustic beam signal from the beam-region acoustic beam signal generated in synchronization with the transmission event and synthesizes the first frame acoustic beam signal from the generated subframe acoustic beam signal. Fig. 12A is a functional block diagram illustrating the configuration of the first synthesizer 1044. As illustrated in Fig. 12A, the first synthesizer 1044 includes an acoustic beam signal development unit 1341 and a weighting synthesizer 1342.

Hereinafter, the configuration of each component constituting the first synthesizer 1044 will be described.

i) Acoustic Beam Signal Development Unit 1341

After the generation of a series of beam-region acoustic beam signals for synthesizing the first frame acoustic beam signal is completed, the acoustic beam signal development unit 1341 reads the plurality of beam-region acoustic beam signals stored in the data storage unit 107. For each of the beam-region acoustic beam signals, the subframe acoustic beam signal is generated from the beam-region acoustic beam signal based on the positional relationship between the observation point Sij and the representative point Qk in the target region Bx.

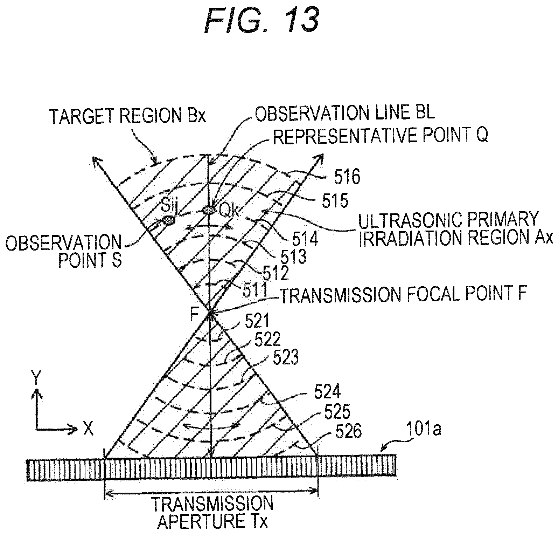

Fig. 13 is a schematic diagram illustrating a process of generating a subframe acoustic beam signal in the acoustic beam signal development unit 1341. First, it is assumed that any one of the depth of the representative point Qk and the depth of the observation point Sij is deeper than the transmission focal depth. As described above, the transmission time of the representative point Qk depends on the sum of the distance from the transmission aperture Tx to the transmission focal point F and the distance from the transmission focal point F to the representative point Qk. That is, in the case where the representative point Qk and the observation point Sij exist at equal distances from the transmission focal point F, the transmission time of the representative point Qk is equal to the transmission time of the observation point Sij. Similarly, the reception time of the representative point Qk depends on the sum of the distance from the observation point Qk to the transmission focal point F and the distance from the transmission focal point F to the reception transducer Rm. That is, in the case where the representative point Qk and the observation point Sij exist at equal distances from the transmission focal point F, the reception time of the representative point Qk is equal to the reception time of the observation point Sij. Therefore, the acoustic beam signals for the representative points Qk include acoustic beam signals for a plurality of observation points Sij having the same distance to the transmission focal point F. In other words, the sum of the acoustic beam signals for a plurality of observation points Sij having the same distance to the transmission focal point F is acquired as an acoustic beam signal for the representative point Qk. This relationship is satisfied even in the case where any one of the depth of the representative point Qk and the depth of the observation point Sij is shallower than the transmission focal depth.

Therefore, the acoustic beam signal development unit 1341 applies the acoustic beam signal of the representative point Qk as the value of the acoustic beam signal of the observation point Sij satisfying the two conditions of (1) both of the depth of the representative point Qk and the depth of the observation point Sij are deeper or shallower than the transmission focal depth and (2) the distance between the representative point Qk and the transmission focal point F is equal to the distance between the observation point Sij and the transmission focal point F. More specifically, the acoustic beam signal development unit sets an arc passing through the representative point Qk within the target region Bx with the transmission focal point F as the center, and for all the observation points Sij existing on the arc, the value of the acoustic beam signal of the representative point Qk existing on the arc is applied as the value of the acoustic beam signal corresponding to the observation point Sij. At this time, arcs which are not continuous in the target region Bx, that is, an arc having a distance from the transmission focal point F but being shallower than the focal depth and an arc being deeper than the focal depth, for example, the arc 521 and the arc 511 are treated as different arcs. For example, for all the observation points Sij on the arc 514, the values of the acoustic beam signal of the representative points Qk existing in the circular arc 514 are applied as the values of the acoustic beam signals corresponding to the observation points Sij. Through such a process, the acoustic beam signal development unit 1341 generates a subframe acoustic beam signal from the beam-region acoustic beam signal.

The acoustic beam signal development unit 1341 outputs the generated subframe acoustic beam signal to the weighting synthesizer 1342.

ii) Weighting Synthesizer 1342

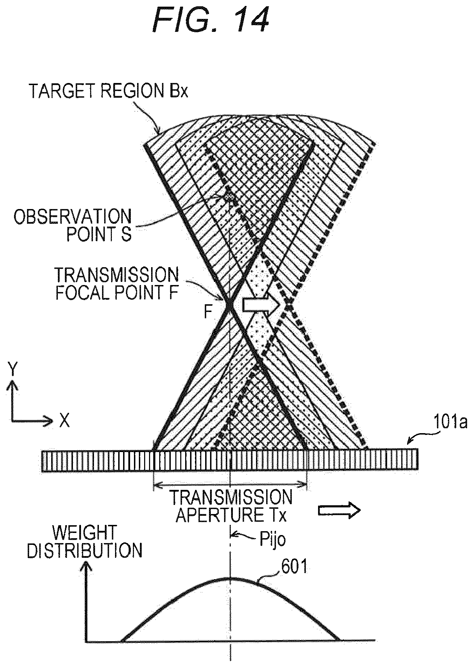

Fig. 14 is a schematic diagram illustrating a process of synthesizing synthesized acoustic beam signals in the weighting synthesizer 1342. As described above, the ultrasonic wave transmission/reception is sequentially performed by allowing the transducers used for the transmission transducer column (transmission aperture Tx) different in the direction of the transducer column to differ by the moving pitch Mp in synchronization with the transmission event. Therefore, the position of the target region Bx based on different transmission events also differs by the moving pitch Mp in the same direction for each transmission event. By adding a plurality of subframe acoustic beam signals with the position of the observation point Sij corresponding to the acoustic beam signal included in each subframe acoustic beam signal as an index, the first frame acoustic beam signal covering all the target regions Bx is synthesized.

At this time, the weighting synthesizer 1342 performs weighting the subframe acoustic beam signal with the position of the observation point Sij as an index. The weight series is a sequence of weight coefficients applied to each subframe acoustic beam signal corresponding to the observation point Sij. The weight series is defined by the position of the transmission focal point F in the transmission event corresponding to the subframe acoustic beam signal. The weight series has a symmetric distribution centered on the observation point Sij. The weight series is set so that the weight for the subframe acoustic beam signal at the transmission event in which the transmission focal point F is set at the same X coordinate (position in the transducer arrangement direction) as the observation point Sij is maximized. The central axis of the weight distribution coincides with the straight line Pijo passing through the observation point Pij and perpendicular to the transducer column. The shape of the weight series is, for example, a Hamming window, a Hanning window, or a rectangular window.

The weighting synthesizer 1342 synthesizes the first frame acoustic beam signal by weighting and adding the subframe acoustic beam signals corresponding to the observation points Pij for the observation points Pij.

In addition, with respect to the observation points Pij existing across the plurality of target regions Bx having different positions, the values of the acoustic beam signals in the respective subframe acoustic beam signals are added, so that the synthesized acoustic beam signal exhibits a large value according to the extent of spreading. Hereinafter, the number of times that the observation point Pij is included in the different target regions Bx is referred to as a "number of times of superposition", and the maximum value of the number of times of superimposition in the direction of the transducer column is referred to as a "maximum number of times of superimposition".

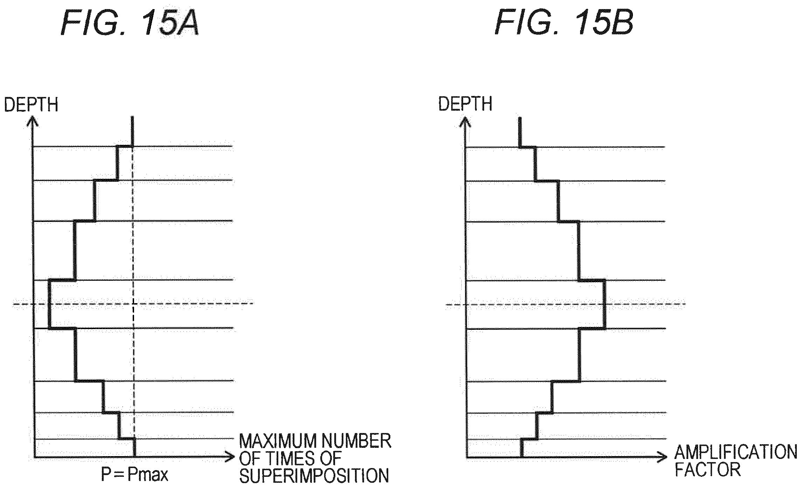

In addition, in this embodiment, the target region Bx exists within the hourglass-shaped area. Therefore, as illustrated in Fig. 15A, since the number of times of superimposition and the maximum number of times of superimposition change in the depth direction of the subject, the value of the synthesized acoustic beam signal also changes in the depth direction. In order to compensate for the change, the weighting synthesizer 1342 performs an amplification process for multiplying each synthesized acoustic beam signal by an amplification factor determined according to the number of times of addition in synthesis of the synthesized acoustic beam signal included in the frame acoustic beam signal.

Fig. 15B is a schematic diagram illustrating an outline of the amplification process in the weighting synthesizer 1342. As illustrated in Fig. 15B, since the maximum number of times of superimposition changes in the depth direction of the subject, the amplification factor that changes in the depth direction of the subject determined according to the maximum number of times of superimposition is multiplied with the synthesized acoustic beam signal so as to compensate for the change. As a result, the variation factor of the synthesized acoustic beam signal due to the change in the number of times of superimposition in the depth direction is eliminated, and the value of the synthesized acoustic beam signal after the amplification process is allowed to be uniform in the depth direction.

In addition, the process of multiplying the synthesized acoustic beam signal by an amplification factor that changes in the direction of the transducer column determined according to the number of times of superposition may be performed. In the case where the number of times of superimposition changes in the direction of the transducer column, the variation factor is eliminated, and the value of the synthesized acoustic beam signal after the amplification process is allowed to be uniform in direction of the transducer column.

In addition, the signal obtained by performing the amplification process on the synthesized acoustic beam signal for each generated observation point may be used as a frame acoustic beam signal.

(6) Second Synthesizer

The second synthesizer 1045 is a circuit that synthesizes a frame acoustic beam signal from a subframe acoustic beam signal generated in synchronization with a transmission event. Fig. 12B is a functional block diagram illustrating a configuration of the second synthesizer 1045. As illustrated in Fig. 12B, the synthesizer 1045 includes an addition processing unit 1343 and an amplification processing unit 1344.

Hereinafter, the configuration of each component constituting the second synthesizer 1045 will be described.

i) Addition Processing Unit 1343

After the generation of a series of subframe acoustic beam signals for synthesizing the frame acoustic beam signals is completed, the addition processing unit 1343 reads the plurality of subframe acoustic beam signals stored in the data storage unit 107. Next, by adding a plurality of subframe acoustic beam signals with the position of the observation point Pij where the acoustic beam signal included in each subframe acoustic beam signal is acquired as an index, a synthesized acoustic beam signal for each observation point is generated, so that frame acoustic beam signal is synthesized. Therefore, the acoustic beam signals for the observation points at the same position included in the plurality of subframe acoustic beam signals are added to generate a synthesized acoustic beam signal.

Similarly to the weighting synthesizer 1342, the addition processing unit 1343 synthesizes the second frame acoustic beam signal covering all the target regions Cx by adding a plurality of subframe acoustic beam signals with the position of the observation point Pij where the acoustic beam signal included in each subframe acoustic beam signal is acquired as an index.

ii) Amplification Processing Unit 1344

As described above, the value of the synthesized acoustic beam signal changes in the depth direction of the subject. In order to compensate for the change, the amplification processing unit 1344 performs an amplification process for multiplying each synthesized acoustic beam signal by an amplification factor determined according to the number of times of addition in synthesis of the synthesized acoustic beam signal included in the frame acoustic beam signal. The amplification processing unit 1344 performs an amplification process similar to that of the weighting synthesizer 1342. As illustrated in Fig. 15B, since the maximum number of times of superimposition changes in the depth direction of the subject, the amplification factor that changes in the depth direction of the subject determined according to the maximum number of times of superimposition is multiplied with the synthesized acoustic beam signal. As a result, the variation factor of the synthesized acoustic beam signal due to the change in the number of times of superimposition in the depth direction is eliminated, and the value of the synthesized acoustic beam signal after the amplification process is allowed to be uniform in the depth direction.

(7) Output Unit

The output unit 1046 is a selector circuit that outputs any one of the frame acoustic beam signal generated by the first synthesizer 1044 and the frame acoustic beam signal generated by the second synthesizer 1045 to the ultrasonic image generator 105 under the control of the controller 108. In the case of using the high-performance arithmetic circuit 1047, the controller 108 allows the output unit 1046 to output the frame acoustic beam signal generated by the second synthesizer 1045. On the other hand, in the case of not using the high-performance arithmetic circuit 1047, the controller 108 allows the output unit 1046 to output the frame acoustic beam signal generated by the first synthesizer 1044.

<Operation 1: In Case of Using Second Phasing Adder>

The operation of the ultrasonic diagnostic apparatus 100 having the above configuration will be described.

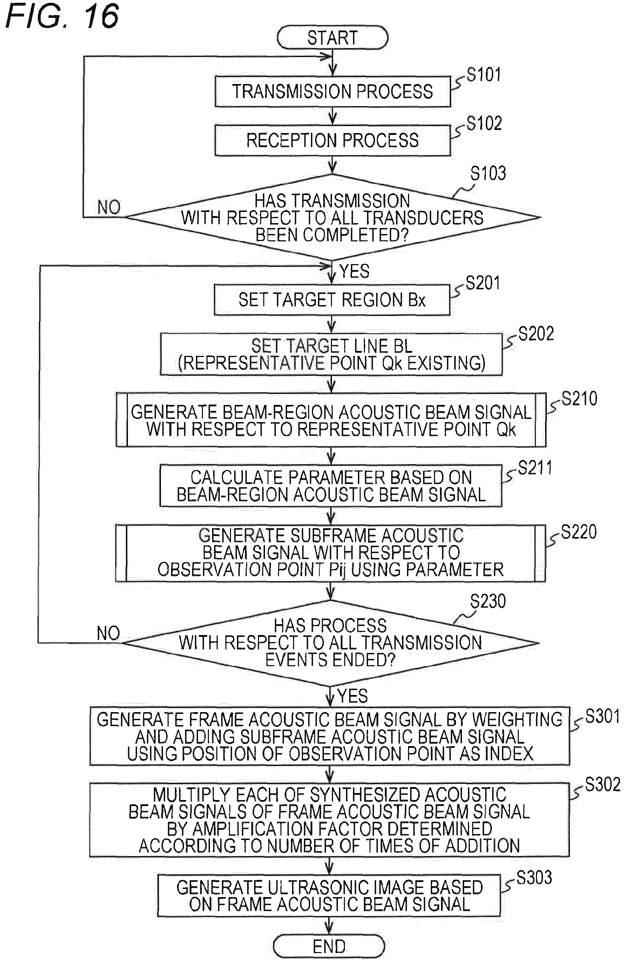

Fig. 16 is a flowchart illustrating a beamforming processing operation of the reception beamformer unit 104 in the case of using the second phasing adder.

First, in step S101, the transmitter 1031 performs a transmission process (transmission event) for supplying a transmission signal for transmitting an ultrasonic beam to each of the transducers included in the transmission aperture Tx among the plurality of transducers 101a existing in the probe 101.

Next, in step S102, the receiver 1040 generates a reception signal based on the electric signal obtained from the reception of the reflected ultrasonic wave by the probe 101, outputs the reception signal to the data storage unit 107, and stores the reception signal to the data storage unit 107. It is determined whether the ultrasonic wave transmission has been completed from all the transducers 101a existing in the probe 101 (step S103). Then, in the case where the ultrasonic wave transmission has not been completed, the process returns to step S101, and the transmission event is performed while moving the transmission aperture Tx by the moving pitch Mp in the column direction. In the case where the ultrasonic wave transmission has been completed, the process proceeds to step S201.

Next, in step S201, the target region setting unit 1141 sets the target region Bx based on the information indicating the position of the transmission aperture Tx in synchronization with the transmission event. In the first loop, the target region Bx obtained from the transmission aperture Tx in the initial transmission event is set.

Next, in step S202, the target region setting unit 1141 sets the target line BL in the set target region Bx. The target line BL exists inside the target region Bx and is a straight line region passing through the transmission focal point F.

Next, in step S210, an acoustic beam signal is generated with respect to the representative point Qk.

Herein, an acoustic beam signal generation operation for the representative point Qk in step S210 will be described. In the following description, the operation of generating only one acoustic beam signal with respect to the representative point Qk will be described. However, as described above, while changing only the parameter of the ultrasonic velocity, a plurality of acoustic beam signals is generated with respect to the same representative point Qk. That is, while only the value of the ultrasonic velocity is changed, step S210 is executed several times.

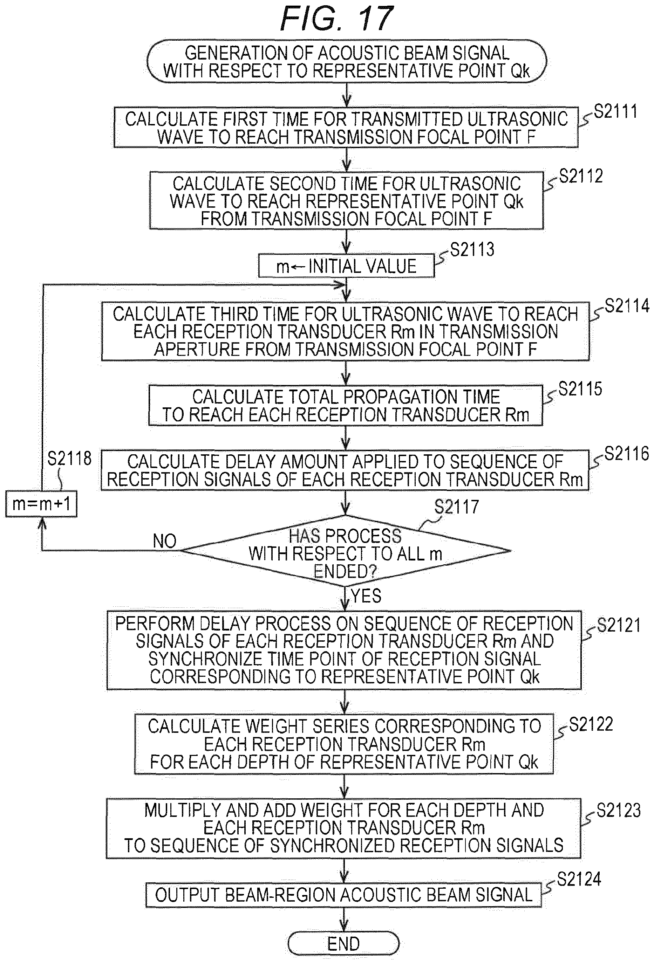

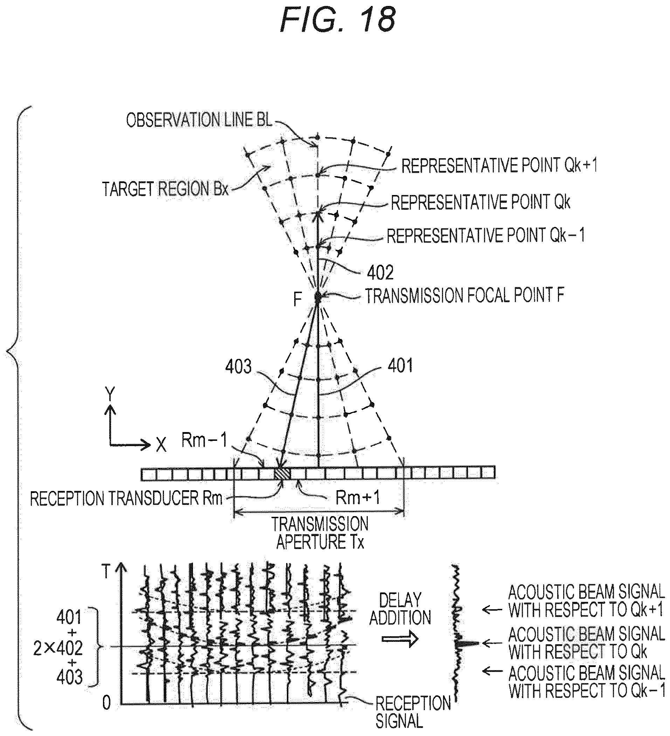

Fig. 17 is a flowchart illustrating an acoustic beam signal generation operation with respect to the representative point Qk in the reception beamformer unit 104. Fig. 18 is a schematic diagram illustrating the acoustic beam signal generation operation on the representative point Qk in the reception beamformer unit 104.

First, in step S2111, the transmission time calculator 1142 calculates a first time for the transmitted ultrasonic wave to reach the transmission focal point F. The first time can be calculated by dividing the length of the route (401) from the geometrically determined transmission aperture Tx to the transmission focal point F by the sound velocity cs of the ultrasonic wave.

Next, in step S2112, the transmission time calculator 1142 calculates a second time to reach the representative point Qk from the transmission focal point F. The second time can be calculated by dividing the length of the route (402) from the geometrically determined transmission focal point F to the representative point Qk by the sound velocity cs of the ultrasonic wave. In the case where the depth of the representative point Qk is shallower than the transmission focal depth, a negative value with the calculated value as an absolute value is taken as the second time. That is, assuming that the second time of the representative point Qx of which depth is deeper than the transmission focal depth is 1.5 $\mu$s with respect to the two representative points Qx and Qy equidistant from the transmission focal point, the second time of the representative point Qy of which depth is shallower than the transmission focal depth is set to -1.5 $\mu$s.

The transmission time calculator 1142 outputs the sum of the first time and the second time as the transmission time for the representative point Qk to the delay amount calculator 1144 and outputs the second time to the reception time calculator 1143.

Next, the transmission time calculator initializes the coordinate m indicating the position of the reception transducer Rm obtained from the transmission aperture Tx to the minimum value in the transmission aperture Tx (step S2114), and calculates the reception time where the transmitted ultrasonic wave is reflected by the representative point Qk in the subject and reaches the reception transducer Rm of the transmission aperture Tx. Herein, the time when the ultrasonic wave reflected at the representative point Qk reaches the transmission focal point F has already been calculated as the second time in step S2112. Therefore, the reception time calculator 1143 calculates a third time when the reflected ultrasonic wave reaches the reception transducer Rm of the transmission aperture Tx from the transmission focal point F (step S2114). The third time can be calculated by dividing the length of the route 403 from the geometrically determined transmission focal point F to the reception transducer Rm by the sound velocity cs of the ultrasonic wave. Then, the reception time calculator 1143 outputs a sum of the second time and the third time as the reception time to the delay amount calculator 1144. In addition, the delay amount calculator 1144 calculates the total propagation time until the ultrasonic wave transmitted from the transmission aperture Tx is reflected at the representative point Qk and reaches the reception transducer Rm based on the sum of the transmission time and the reception time (step S2115) and calculates the delay amount for each of the reception transducers Rm based on the difference in the total propagation time for each of the reception transducers Rm in the transmission aperture Tx (step S2116).

It is determined whether the calculation of the delay amount has been completed with respect to all the reception transducers Rm existing in the transmission aperture Tx (step S2117). In the case where the calculation of the delay amount has not been completed, the coordinate m is incremented (step S2118), and in addition, the calculation of the delay amount of the reception transducer Rm is performed (step S2114). In the case where the calculation of the delay amount has been completed, the process proceeds to step S2121. At this stage, the delay amount of arrival of the reflected wave from the representative point Qk is calculated with respect to all of the reception transducers Rm existing in the transmission aperture Tx.

In step S2121, the delay processing unit 1145 performs a delay process on a sequence of reception signals corresponding to the reception transducers Rm in the transmission aperture Tx based on the delay amount for each of the reception transducers Rm and synchronizes the time point (timing) of the reception signal based on the reflected wave from the representative point Qk. As described above, among the total propagation time, the first time is uniquely determined by the positional relationship between the transmission focal point F and the transmission aperture Tx, the second time is uniquely determined by the positional relationship between the transmission focal point F and the representative point Qk, and the third time is uniquely determined by the positional relationship between the transmission focal point F and the reception transducer Rm. Herein, since any one of the position of the transmission focal point F and the position of the transmission aperture Tx is constant in one transmission event, the first time is constant for all the representative points Qk and all the reception transducers Rm. In addition, since the second time does not depend on the position of the reception transducer Rm, the difference in the total propagation time between the representative point Qk and the representative point Q(k+1) in one reception transducer Rm does not depend on the coordinate m. That is, the time difference between the reception signal based on the reflected wave from the representative point Qk and the reception signal based on the reflected wave from the representative point Q(k+1) in a sequence of the reception signals corresponding to the same reception transducer depends only on the distance between the representative point Qk and the representative point Q(k+1), and there is no difference between the signal corresponding to the reception transducer Rm and the signal corresponding to the reception transducer R(m+1). On the other hand, since the third time does not depend on the position of the representative point Qk, the time difference between the reception signal based on the reflected wave from the representative point Qk in a sequence of reception signals corresponding to the reception transducer Rm and the reception signal based on the reflected wave from the representative point Qk in a sequence of the reception signals corresponding to the reception transducer R(m+1) depends only on the positional relationships of three positions of the reception transducer Rm, the reception transducer R(m+1), and the transmission focal point F, and there is no difference between the reception signal based on the reflected wave from the representative point Qk and the reception signal based on the reflected wave from the representative point Q(k+1). Therefore, if the delay process for canceling the difference in the third time with respect to each sequence of the reception signals corresponding to the each reception transducer Rm is performed, the time points of the reception signals based on the reflected wave from the representative point Qk are aligned between the sequences of reception signals, and the time points of the reception signals based on the reflected wave from the representative point Q(k+1) and the time points of the reception signals based on the reflected wave from the representative point Q(k-1) are aligned. Therefore, it is unnecessary to identify the reception signals based on the total propagation time for each representative point Qk, and it is possible to identify the reception signal for each representative point Qk based on the first time and the second time as the sequence of reception signals in the direction of the transducer column by performing the delay process based on the third time is performed on the sequence of reception signals.

Next, the weight calculator 1146 calculates a weight series for each of the reception transducers Rm (step S2123). For example, the weight calculator 1146 applies a Hamming window, a Hanning window, a rectangular window. The adder 1147 generates an acoustic beam signal for the representative point Qk by multiplying and adding the weight for each of the reception transducers Rm to a sequence of reception signals identified corresponding to each of the reception transducers Rm (step S2123), and the generated acoustic beam signal corresponding to the representative point Qk is output to the data storage unit 107 for storage (step S2124).

Next, returning to Fig. 16, in step S211, the parameter calculator 1043 calculates a parameter based on the beam-region acoustic beam signal. Herein, with respect to each of a plurality of beam-region acoustic beam signals generated for the same target region BL, the turbulence (arithmetic mean of square of the values of the acoustic beam signals) of the values of the acoustic beam signals is calculated. Then, the ultrasonic velocity corresponding to the acoustic beam signal with the largest turbulence is calculated as the ultrasonic velocity in the subject. The calculated ultrasonic velocity is output to the second phasing adder 1042.

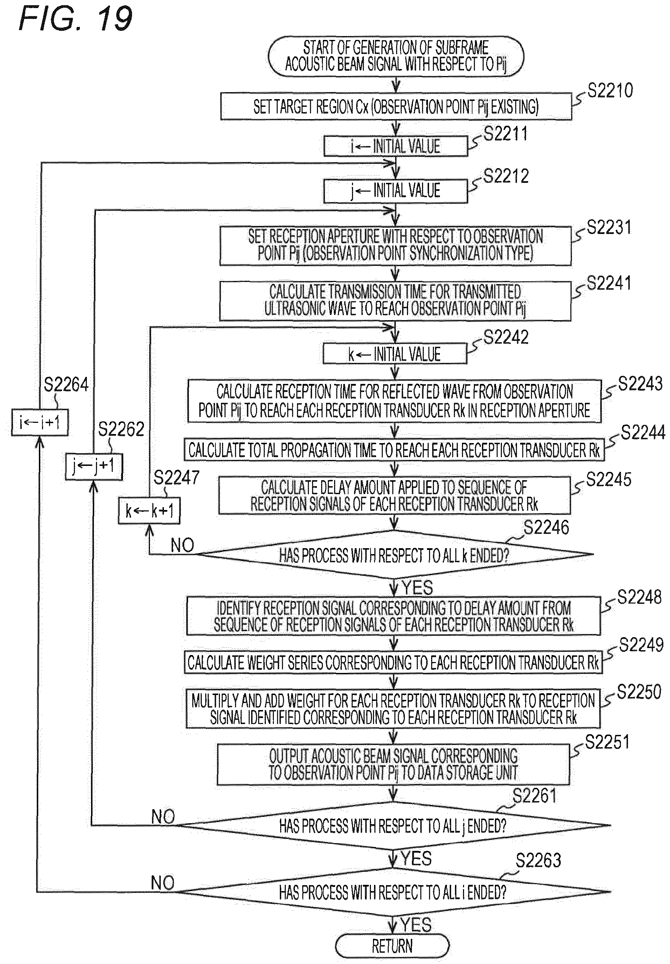

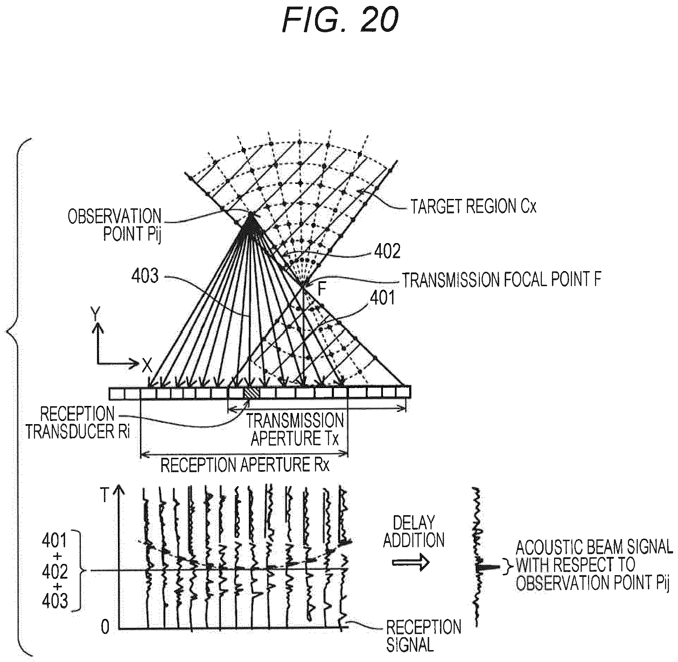

Next, in step S220, the acoustic beam signals are generated for the observation points Pij by using the parameter. Fig. 19 is a flowchart illustrating an acoustic beam signal generation operation for the observation point Pij in the reception beamformer unit 104. Fig. 20 is a schematic diagram illustrating the acoustic beam signal generation operation on the observation point Pij in the reception beamformer unit 104.

First, in step S2210, the target region setting unit 1241 sets the target region Cx that is the setting range of the observation point Pij. Herein, the entire target region Bx is set as the target region Cx.

Next, the process proceeds to the observation point synchronized beamforming processes (steps S2211 to 2264). First, the coordinate ij indicating the position of the observation point Pij is initialized to the minimum value of the target region Cx (steps S2211 and S2212), and the reception aperture setting unit 1242 selects the transducer column in the reception aperture Rx so that the center of the column coincides with the transducer Xk that is spatially closest to the observation point Pij (step S2231).

Next, an acoustic beam signal is generated for the observation point Pij.

First, in step S2241, the transmission time calculator 1243 calculates the transmission time when the transmitted ultrasonic wave reaches the observation point Pij in the subject with respect to an arbitrary observation point Pij existing on the target region Cx. At this time, the transmission time calculator 1243 calculates the transmission time by using the geometric route length determined by the route 401 and the route 402 and the velocity of the ultrasonic wave calculated by the parameter calculator 1043.

Next, the coordinate k indicating the position of the reception transducer Rk in the reception aperture Rx obtained from the reception aperture Rx is initialized to the minimum value in the reception aperture Rx (step S2242), and the reception time when the transmitted ultrasonic wave is reflected at the observation point Pij in the subject and reaches the reception transducer Rk of the reception aperture Rx is calculated (step S2243). The reception time can be calculated by dividing the length of the route 403 from the observation point Pij geometrically determined to the reception transducer Rk by the velocity of the ultrasonic wave calculated by the parameter calculator 1043. In addition, the total propagation time until the ultrasonic wave transmitted from the transmission aperture Tx is reflected at the observation point Pij and reaches the reception transducer Rk is calculated from the sum of the transmission time and the reception time (step S2244), and the delay amount for each of the reception transducers Rk is calculated from the difference in the total propagation time for each of the reception transducers Rk in the reception aperture Rx (step S2245).

It is determined whether the calculation of the delay amount has been completed with respect to all the reception transducers Rk existing in the reception aperture Rx (step S2246). In the case where the calculation of the delay amount has not been completed, the coordinate k is incremented (step S2247), and in addition, the calculation of the delay amount of the reception transducer Rk is performed (step S2243). In the case where the calculation of the delay amount has been completed, the process proceeds to step S2248. At this stage, the delay amount of arrival of the reflected wave from the observation point Pij is calculated for all of the reception transducers Rk existing in the reception aperture Rx.

In step S2248, the delay processing unit 1145 identifies the reception signal corresponding to the time obtained by subtracting the delay amount for each of the reception transducers Rk from a sequence of reception signals corresponding to the reception transducers Rk in the reception aperture Rx as the reception signal based on the reflected wave from the observation point Pij.

Next, the weight calculator 1247 calculates a weight series for each of the reception transducers Rk so that the weight for the transducer located at the center of the reception aperture Rx in the column direction is maximized (step S2249). The adder 1248 generates an acoustic beam signal for the observation point Pij by multiplying and adding the weight for each of the reception transducers Rk to the reception signal identified corresponding to each of the reception transducers Rk (step S2250), and the generated acoustic beam signal for the observation point Pij is output to the data storage unit 107 for storage (step S2251).

Next, by incrementing the coordinate ij and repeating steps S2231 to S2251, the acoustic beam signals are generated for all the observation points Pij ("·" in Fig. 20) located at the coordinates ij in the target region Bx. It is determined whether the generation of acoustic beam signals has been completed for all the observation points Pij existing in the target region Cx (steps S2261 and S2263). In the case where the generation of acoustic beam signals has not been completed, the coordinate ij is incremented (steps S2262 and S2264), the acoustic beam signal for the observation point Pij is generated. In the case where the generation of acoustic beam signals has been completed, the process proceeds to step S230. At this stage, acoustic beam signals of subframes for all the observation points Pij existing in the target region Cx associated with one transmission event are generated and output to the data storage unit 107 for storage.

Next, for all the transmission events, it is determined whether the generation of the acoustic beam signal of the subframe has ended (step S230). In the case where the generation of the acoustic beam signal has not been ended, the process returns to step S210. In the case where the generation of the acoustic beam signal has been ended, the process proceeds to step S301.

Next, in step S301, the addition processing unit 1343 reads a plurality of subframe acoustic beam signals stored in the data storage unit 107 and generates synthesized acoustic beam signals for each observation point Pij to synthesize frame acoustic beam signals by adding the plurality of subframe acoustic beam signals with the position of the observation point Pij as an index. Next, the amplification processing unit 1344 multiplies each of the synthesized acoustic beam signals by an amplification factor determined according to the number of times of addition of each synthesized acoustic beam signal included in the frame acoustic beam signal (step S302), and the amplified frame acoustic beam signal is output to the ultrasonic image generator 105 and the data storage unit 107 (step S303), and the process is ended.

<Operation 2: Case of Not Using Second Phasing Adder>

On the other hand, the operation of the ultrasonic diagnostic apparatus 100 in the case of not using the second phasing adder 1042 will be described.

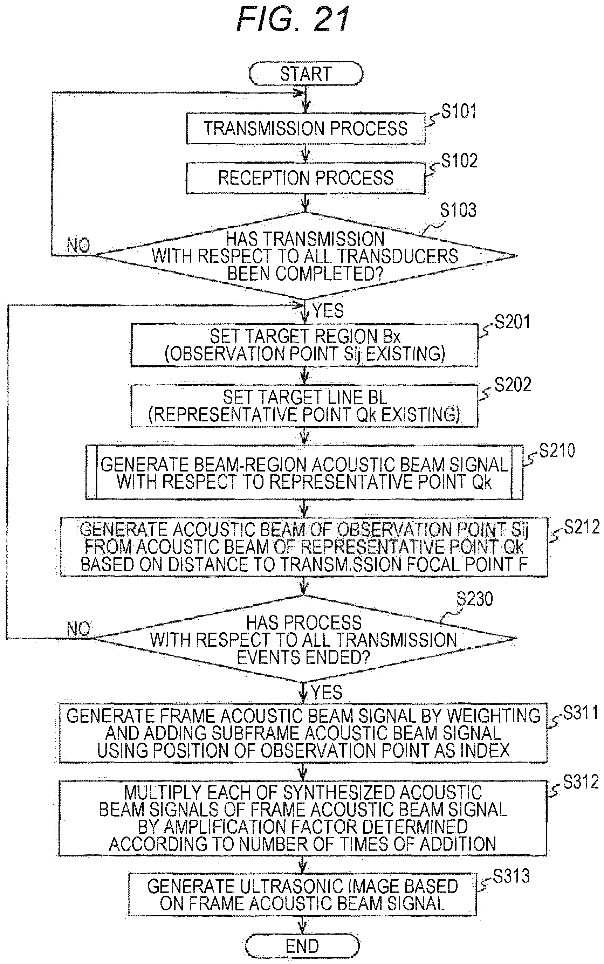

Fig. 21 is a flowchart illustrating a beamforming process operation of the reception beamformer unit 104 in the case of not using the second phasing adder. In addition, the same step numbers are denoted to the same operations as in the case of using the second phasing adder illustrated in Fig. 16, and detailed description thereof will be omitted.

First, in step S101, the transmitter 1031 performs a transmission process (transmission event) for supplying a transmission signal for transmitting an ultrasonic beam to each of the transducers included in the transmission aperture Tx among the plurality of transducers 101a existing in the probe 101.

Next, in step S102, the receiver 1040 generates a reception signal based on the reflected ultrasonic wave in the probe 101, outputs the reception signal to the data storage unit 107, and stores the reception signal in the data storage unit 107. It is determined whether the ultrasonic wave transmission has been completed from all of the transducers 101a existing in the probe 101 (step S103). In the case where the ultrasonic wave transmission has not been completed, the process returns to step S101, and the transmission event is performed while moving the transmission aperture Tx by the moving pitch Mp in the column direction. In the case where the ultrasonic wave transmission has been completed, the process proceeds to step S201.

Next, in step S201, the target region setting unit 1141 sets a target region Bx based on the information indicating the position of the transmission aperture Tx in synchronization with the transmission event.

Next, in step S202, the target region setting unit 1141 sets a target line BL in the set target region Bx.

Next, in step S210, an acoustic beam signal is generated with respect to the representative point Qk.

Next, in step S212, the acoustic beam signal development unit 1341 generates a subframe acoustic beam signal for the observation point Sij in the target region Bx based on a beam-region acoustic beam signal. As described above, in the case where the observation point Sij is deeper than the transmission focal depth among the representative points Qk having the same distance to the transmission focal point F with respect to the observation point Sij, the acoustic beam signal development unit 1341 specifies the representative point Qk that is deeper than the transmission focal depth, and in the case where the observation point Sij is shallower than the transmission focal depth, the acoustic beam signal development unit specifies the representative point Qk that is shallower than the transmission focal depth. Then, the acoustic beam signal development unit sets the acoustic beam signal for the specified representative point Qk as an acoustic beam signal for the observation point Sij. At this stage, acoustic beam signals of subframes for all observation points Sij existing in the target region Bx associated with one transmission event are generated.

Next, with respect all the transmission events, it is determined whether the generation of the acoustic beam signal of the subframe has been ended (step S230). In the case where the generation of the acoustic beam signal has not been ended, the process returns to step S201, and the target region Bx is set based on the transmission aperture Tx (step S201). In the case where the generation of the acoustic beam signal has been ended, the process proceeds to step S311.

Next, in step S311, the weighting synthesizer 1342 sets a weight series for the subframe acoustic beam signal based on the position of the transmission focal point F in the transmission event corresponding to the subframe acoustic beam signal for the observation point Pij.

Next, in step S312, the weighting synthesizer 1342 weights and adds the plurality of subframe acoustic beam signals with the position of the observation point Pij as an index by using the weight series, generates a synthesized acoustic beam signal for each observation point Pij, and synthesizes the frame acoustic beam signals. Next, the weighting synthesizer 1342 outputs the frame acoustic beam signal to the ultrasonic image generator 105 and the data storage unit 107 (step S303), and the process is ended.

<Summary>

As described above, according to the ultrasonic diagnostic apparatus 100 according to this embodiment, the acoustic beam signals of observation points at the same position generated by different transmission events are superimposed to be synthesized by the synthetic aperture method. As a result, even at observation points in depths other than the transmission focal point F for a plurality of transmission events, the effects of virtually performing the transmission focus can be obtained, and the spatial resolution and the signal S/N ratio can be improved.

In addition, in the ultrasonic diagnostic apparatus 100, when generating a beam-region acoustic beam signal, the phasing addition is not performed on all the observation points Pij, but in each of the arc regions centered on the transmission focal point F, one representative point Qk is provided, and the phasing addition is performed only on the representative point Qk. Thus, since the number of representative points Qk to be subjected to the phasing addition depends not on the area of the target region Bx but on the length in the depth direction of the target region Bx, it is possible to greatly reduce the calculation amount of the phasing addition. Furthermore, not only the transmission time but also the reception time is set based on the transmission focal point F, so that it is not necessary to repeat the reception time calculation process for each of the reception transducers Rm for each representative point Qk. Therefore, both the calculation process of the total propagation time and the phasing addition process can be simplified, and also in this point, it is possible to greatly reduce the calculation amount of the phasing addition. On the other hand, since it is possible to obtain the effect of improving the spatial resolution and the signal S/N ratio by synthesizing different subframe acoustic beam signals for the same observation point, the decrease in the spatial resolution and the signal S/N ratio can be suppressed relative to the degree of decrease in the calculation amount.

Furthermore, in the ultrasonic diagnostic apparatus 100, in the case of using the second phasing adder 1042, a parameter such as an ultrasonic velocity is calculated based on the beam-region acoustic beam signal, and the parameter is reflected to generate the subframe acoustic beam signal. Thus, in the case of changing the parameter of the phasing addition based on the acoustic beam signal generated by the phasing addition, it is not necessary to repeat the generation of the subframe acoustic beam signal having a large calculation amount. Furthermore, since the beam-region acoustic beam signal used for the evaluation is generated independently of the subframe acoustic beam signal, it is possible to distribute the calculation ability of the second phasing adder and the second synthesizer only to generation of the frame acoustic beam signal. Therefore, it is possible to adjust the parameter while maximizing the calculation ability of the second phasing adder and the second synthesizer.

In addition, in the ultrasonic diagnostic apparatus 100, in the case of not using the second phasing adder 1042, a frame acoustic beam signal is generated based on a beam-region acoustic beam signal. Therefore, it is possible to generate the frame acoustic beam signal with a low calculation amount appropriate for the calculation ability of the first phasing adder and the first synthesizer. For this reason, even in the case where the calculation ability of the first phasing adder is low, it is possible to generate an ultrasonic image while suppressing a decrease in spatial resolution and signal S/N ratio.

<Modified Example 1>

In the ultrasonic diagnostic apparatus 100 according to the first embodiment, the parameter calculator 1043 calculates the velocity of ultrasonic waves. However, the parameter calculated by the parameter calculator is not limited to the ultrasonic velocity, but an arbitrary parameter that can be optimized afterward from the acoustic beam signal may be employed.

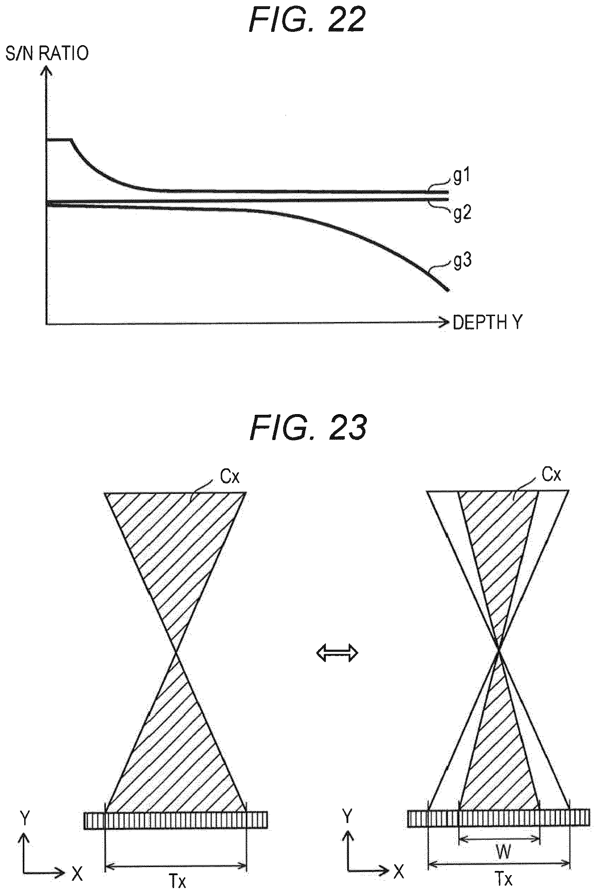

Another specific example of the parameter is, for example, a gain correction value. Before the A/D conversion, the receiver performs a "gain control" process of amplifying the electric signal obtained by receiving the reflected ultrasonic wave in each of the transducers 101a of the probe 101. Since the level of the quantization noise mixed in by the A/D conversion is constant, in order to minimize the influence of the quantization noise, it is preferable that the amplitudes of the amplified electric signals are large within a range not exceeding the maximum value and are aligned. However, in the case where the electric signal is weak, particularly, in the case where white noise or the like is mixed in, excessive amplification allows the white noise and the like to be amplified at the same time, and noise is mixed in the acoustic beam signal after the phasing addition, so that there is a problem of decreasing the quality. Therefore, in the gain control, it is preferable to amplify so that the S/N ratio becomes constant. However, since the ultrasonic attenuation factor in the subject is influenced by the hardness of the tissue, the existence of the boundary of the tissue and the like, it is not necessarily performed properly. Therefore, the relationship between the depth of the observation point and the S/N ratio is calculated from the beam-region acoustic beam signal and amplification is performed according to the depth of the observation point in order to minimize the change in the S/N ratio with respect to the depth. The amplification factor is a gain correction value. More specifically, similarly to the ultrasonic velocity, a plurality of gain correction value distributions which are weight coefficients for the depth of the observation point is provided, and a plurality of beam-region acoustic beam signal is generated for the same target regions BL of which only the gain correction values are different, and as illustrated in Fig. 22, the gain correction value distribution g2 having the smallest change in S/N ratio with respect to the depth can be output to the second phasing adder. Therefore, it is possible to generate a high-quality acoustic beam signal without unevenness in the S/N ratio.

In addition, the parameter may be calculated based on the first frame acoustic beam signal generated by the first synthesizer based on the beam-region acoustic beam signal instead of the beam-region acoustic beam signal. As a specific example of the parameter, for example, there may be exemplified the width of the target region Cx. More specifically, the movement of the tissue and/or the probe in the subject is detected based on the time change of the first frame acoustic beam signal, and as illustrated in Fig. 23, the parameter is calculated so that, as the amount of the movement of the tissue and/or the probe in the subject is increased, the width W of the target region Cx becomes small. The movement of tissue and/or probe in the subject can be detected, for example, by performing a correlation process with the first frame acoustic beam signals of two consecutive frames and determining whether the correlation value is lower than the threshold value. Therefore, it is possible to reduce the number of times of superimposition at the time of moving the probe and to suppress the occurrence of motion artifacts. In addition, for example, the width of the target region Cx may be narrowed in multiple steps according to the tissue in the subject and/or the size of the movement of the probe. More specifically, when the correlation value is lower than a first threshold value, the width W of the target region Cx may be set to 1/2 of the width Tx of the target region Bx, and when the correlation value is lower than a second threshold value, the width W of the target region Cx may be set to 1/3 of the width Tx of the target region Bx.

In addition, the parameters are not limited to the above example and may be an arbitrary parameter that can be calculated from the beam-region acoustic beam signal or the first frame acoustic beam signal. In addition, the ultrasonic velocity and the gain correction value may be calculated from the first frame acoustic beam signal.

<Second Embodiment

In the first embodiment and the modified example, the case where the configuration of the ultrasonic diagnostic apparatus does not change depending on whether the second phasing adder is used has been described. However, the high-performance arithmetic circuit constituting the second phasing adder and the second synthesizer is detachable, so that the ultrasonic diagnostic apparatus may use the second phasing adder only when the high-performance arithmetic circuit is mounted.

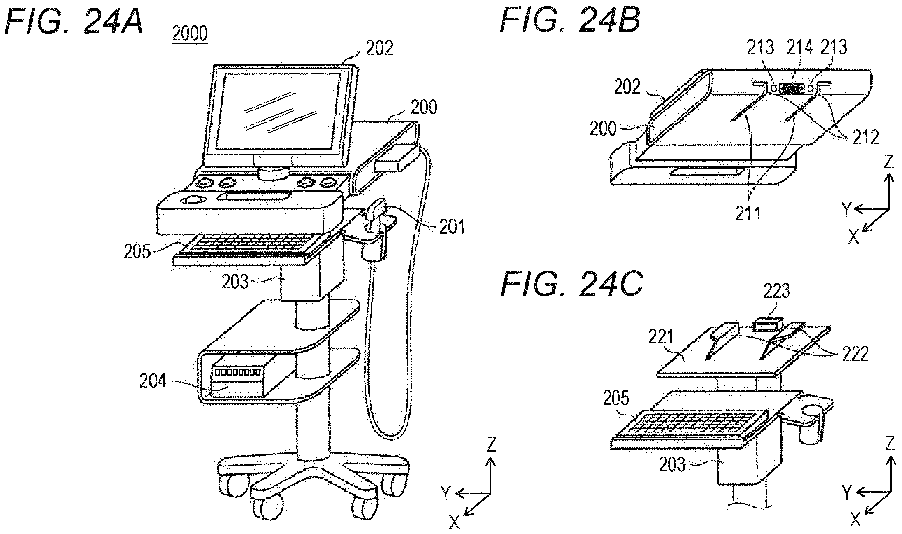

Fig. 24A is an outer appearance view illustrating an ultrasonic diagnostic system 2000 according to a second embodiment. As illustrated in Fig. 24A, the ultrasonic diagnostic system 2000 includes a portable main body (hand carry unit (HCU)) 200, a probe 201, a cart 203, an extension unit 204, and an input unit 205. The display unit 202 is incorporated into the portable main body 200.

The portable main body 200 is an ultrasonic diagnostic apparatus that allows the probe 201 to transmit and receive ultrasonic waves and generates an ultrasonic image based on an output signal from the probe 101 and displays it on the display unit 202, and has an entire configuration of the ultrasonic diagnostic apparatus 100 excluding the high-performance arithmetic circuit 1047, that is, the second phasing adder 1042 and the second synthesizer 1045. The portable main body 200 includes, for example, a switch fabric compatible with PCI Express x16, a personal computer connected to the switch fabric, and a receiver 1040 connected to the switch fabric and configured with an FPGA. In addition, the communication specification of the switch fabric is not limited to the PCI Express x16, but an arbitrary bus that has a sufficient speed (band) for communication between the receiver 1040, the personal computer, and the extension unit 204 described later may be used.

The cart 203 is a movable table with the portable main body 200 mounted thereon and includes a top plate 221, the extension unit 204, and the input unit 205.

The extension unit 204 includes a high-performance arithmetic circuit 1047. More specifically, the extension unit is configured with a GPU, a power supply unit for operating the GPU, and the like. In addition, the specific configuration is not limited to the GPU, and the extension unit may be a processor having a high-performance calculation ability or a module including a processor and an arithmetic circuit.

The input unit 205 is, for example, a keyboard connectable to the portable main body 200.

When the portable main body 200 is removed from the cart 203, as illustrated in the flowchart of Fig.

20, the portable main body 200 performs an operation without using the second phasing adder. On the other hand, when the portable main body 200 is attached to the cart 203, as illustrated in the flowchart of Fig. 16, the portable main body 200 performs an operation using the second phasing adder.

<Detachable Configuration>

The back side of the portable main body 200 is provided with a detachable unit. For example, as illustrated in Fig. 24B, the detachable unit includes two engagement grooves 211 extending in the X direction. The engagement groove 211 is an L-shaped groove, and a locking claw 212 is provided on the rear side of the portable main body 200. The locking claw 212 is biased in the engagement groove by a spring, and the back side of the portable main body 200 is an inclined surface. Therefore, it is configured that the insertion of the engagement protrusion 222 described later into the engagement groove 211 is permitted, but the extraction of the engagement protrusion 222 inserted into the inner side of the engagement groove 211 is prevented. A release button 213 for lifting the locking claw 212 from the engagement groove and a connector 214 are provided on the back surface of the portable main body 200. Signal lines extending from the switch fabric are accommodated in the connector 214. It is preferable that the connector 214 corresponds to so-called hot plug insertion (hot plug). In addition, the connector 214 may include a signal line that receives an input from the input unit 205.

On the other hand, the top plate 221 of the cart 203 has a detachable unit. As illustrated in Fig. 24C, for example, two engagement protrusions 222 extending in the X direction and a connector 223 are provided in the detachable unit. The engagement protrusion 222 is an L-shaped protrusion capable of being engaged with the engagement groove 211. As illustrated in Fig. 24C, as the engagement protrusion 222 approaches the front side of the cart 203, the portion extending in the Y direction may become small, or the height in the Z direction may become low in the state where there is no portion extending in the Y direction. With such a shape, the portable main body 200 can move relative to the top plate 221 in the Z direction in the state where only a portion of the engagement protrusion 222 is inserted into the engagement groove 211, the detachment of the portable main body 200 becomes easy. The connector 223 is provided to be engaged with the connector 214 in the state where the engagement protrusion 222 of the top plate 221 is engaged with the engagement groove 211 of the portable main body 200 and the portable main body 200 cannot move relative to the top plate 221 by the locking claw 212. Signal lines extending from the high-performance arithmetic circuit 1047 of the extension unit 204 are accommodated in the connector 223. In addition, the connector 223 may include signal lines from the input unit 205.

When the portable main body 200 is attached to the cart 203, the position of the engagement groove 211 is aligned with the end of the front side of the cart 203 of the engagement protrusion 222, and the portable main body 200 is allowed to slide to the back side of the cart in the X direction. Therefore, the engagement groove 211 serves as a guide, and the engagement protrusion 222 and the engagement groove 211 are engaged with each other. When the engagement protrusion 222 is completely accommodated in the engagement groove 211, the connector 214 and the connector 223 are engaged, and the portable main body 200 and the extension unit 204 are electrically connected to each other. In addition, the locking claw 212 protrudes into the engagement groove 211 and prevents the portable main body 200 from moving relative to the cart 203. Therefore, the portable main body 200 is fixed to the cart 203.

On the other hand, in the detachment of the portable main body 200 from the cart 203, while pushing the release button 213, the portable main body 200 is allowed to slid toward the front side of the cart 203, and the portable main body 200 is further moved to the front side of the cart 203. By pushing the release button 213, the engagement protrusion 222 and the engagement groove 211 can move relative to each other in the X direction, and by sliding the portable main body 200 to the front side of the cart 203, the connection between the connector 214 and the connector 223 is released. Furthermore, by sliding the portable main body 200 to the front side of the cart 203, the engagement between the engagement protrusion 222 and the engagement groove 211 is completely released.

<Functional Configuration>

Figs. 24A to 24C illustrate the configuration of the reception beamformer unit 304 of the portable main body 200. As illustrated in Figs. 24A to 24C, the reception beamformer unit 304 has a configuration excluding the high-performance arithmetic circuit 1047, that is, the second phasing adder 1042 and the second synthesizer 1045 from the reception beamformer unit 104.

The reception beamformer unit 304 performs the operation of <Operation 2: In Case of Not Using second Phasing Adder> by itself. In addition, when the portable main body 200 is fixed to the cart 203, the reception beamformer unit cooperates with the high-performance arithmetic circuit 1047 of the extension unit 204. That is, the reception beamformer unit 104 is implemented by a combination of the reception beamformer unit 304 and the high-performance arithmetic circuit 1047.

The controller of the portable main body 200 determines whether the portable main body 200 is fixed to the cart 203. In the case where the portable main body is not fixed, it is determined not to use the second phasing adder. In the case where the portable main body is fixed, it is determined to use the second phasing adder. As a method of determining whether the portable main body 200 is fixed to the cart 203, for example, there is a method of detecting whether the connection between the switch fabric and the high-performance arithmetic circuit 1047 is valid. In addition, the determination method is not limited to this case, but the controller may attempt communication to the high-performance arithmetic circuit 1047 and the determination may be performed based on whether the communication succeeds. Alternatively, a detection signal line for detecting whether the connector 214 is connected to the connector 223 is provided, and the determination may be performed based on the state of the detection signal line. Alternatively, a sensor for detecting whether the portable main body 200 is fixed to the cart 203 may be provided on the connector 214, the engagement groove 211, or the back surface or the rear surface of the portable main body 200.

<Summary>

According to the ultrasonic diagnostic apparatus according to the second embodiment, in the case of generating an ultrasonic image with only the portable main body, a process of generating a beam-region acoustic beam signal and generating an ultrasonic image from the beam-region acoustic beam signal with a small calculation amount is performed. Therefore, it is unnecessary for the portable main body to have a high calculation ability. In addition, since the power consumption can be reduced by the small amount of computation, miniaturization of the portable main body due to arrangement design of the portable main body, simplification of the circuit, or the like and the extension of the operable time by the built-in battery becomes easy. On the other hand, in the case where the portable main body is mounted on the cart, the high-performance arithmetic circuit generates the ultrasonic image by the synthetic aperture method using the parameter optimized based on the beam-region acoustic beam signal. Therefore, it is possible to optimize a parameter after using the calculation ability of the high-performance arithmetic circuit only for generation of the ultrasonic image, and after generating the high-quality ultrasonic image, it is possible to widen the target region and improve the frame rate. Therefore, even if only the portable main body can be used as an ultrasonic diagnostic apparatus, in the case of being integrated with a cart, the portable main body can be used as a high-quality ultrasonic diagnostic apparatus.

<Other Modified Examples According to Embodiments>

(1) In each of the embodiments and the modified examples, the case where the first phasing adder generates the beam-region acoustic beam signal for the target line BL that is inside the target region Bx and passes through the focal point F, and the first synthesizer generates the first frame acoustic beam signal based on the beam-region acoustic beam signal has been described. However, the reception beamforming of the first phasing adder and the first synthesizer is not limited to the above example. For example, the first phasing adder may generate the beam-region acoustic beam signals by the reception beamforming in the related art with respect to one or a plurality of linear regions BLx passing through the focal point F or the vicinity thereof and being perpendicular to the transducer column 101a. Herein, the reception beamforming in the related art denotes, for example, beamforming in the related art using a value obtained by dividing the shortest distance (that is, the depth of the observation point Q) between the observation point Q and the transmission aperture Tx by the ultrasonic velocity as the transmission time and using a value obtained by dividing the distance between the observation point Q and the reception transducer R by the ultrasonic velocity as the reception time. Even with such a configuration, it is possible to obtain the same effect as the embodiment.

(2) In each of the embodiments and the modified examples, it is assumed that the second phasing adder performs observation point synchronous reception beamforming. However, the second phasing adder may perform transmission aperture synchronous reception beamforming. In this case, the reception aperture is set based on the position of the transmission aperture. More specifically, the reception aperture is provided so that the central axis of the transmission aperture coincides with the transmission axis of the reception aperture. Therefore, the reception aperture does not depend on the position of the observation point but depends on the position of the transmission aperture. For this reason, it is possible to perform the phasing addition at different reception apertures in synchronization with the transmission event, and the reception time differs for a plurality of transmission events, but as a result, it is possible to obtain the effect of the reception process using the wider reception aperture, so that it is possible to obtain the effect of being capable of allowing the spatial resolution to be uniform in a wide observation region.

(3) In the second embodiment, specific shapes of the detachable unit of the portable main body 200 and the cart 203 have been described. However, the configuration of the detachable unit is not limited to the above example. The shape of the detachable unit may be an arbitrary shape if two conditions are satisfied that (a) the portable main body is not separated from the cart against user's attention and that (b) when the portable main body is attached to the cart, the portable main body and the extension unit are electrically connected to each other. It is preferable that it is easy to detach the portable main body from the cart when the user is to detach the portable main body.

In the second embodiment, the connector is provided in each of the portable main body 200 and the cart 203. However, for example, the portable main body and the extension unit may perform wireless communication or non-contact communication.

(4) In each of the embodiments and the modified examples, the case where the second phasing adder performs the phasing addition using the parameter in the transmission event in which the parameter calculator has calculated the parameter has been described. However, it is not necessary that the parameter is reflected in the same transmission event. For example, upon receiving the parameter from the parameter calculator, the second phasing adder may reflect the parameter in subsequent transmission events. With this configuration, the second phasing adder does not need to wait for the generation of the beam-region acoustic beam signal by the first phasing adder and the calculation of the parameter by the parameter calculator, and the phasing addition by the second phasing adder, the generation of the beam-region acoustic beam signal by the first phasing adder, and the calculation of the parameter by the parameter calculator can be performed in parallel processing. Therefore, the calculation ability of the first phasing adder and the parameter calculator does not affect the generation time of the frame acoustic beam signal by the second phasing adder and the second synthesizer, and it is possible to improve the frame rate in the case of using the second phasing adder.

(5) Although the present invention has been described based on the above embodiments, the present invention is not limited to the above-described embodiments, and the following cases are also included in the present invention.

For example, the case where some or all portions of the ultrasonic diagnostic apparatus are configured with a computer system including a microprocessor, a recording medium such as a ROM and a RAM, a hard disk unit and the like is also included in the present invention. A computer program for achieving the same operation as each of the above devices is stored in the RAM or the hard disk unit. The microprocessor operates according to the computer program, so that the function of each device is achieved.

In addition, some or all of components constituting each of the above devices may be configured with one system large scale integration (LSI). The system LSI is a super multifunctional LSI manufactured by integrating a plurality of components into one chip, and specifically, the system LSI is a computer system including a microprocessor, a ROM, a RAM, and the like. These may be separately formed into one chip or may be integrated into one chip so as to include some or all of the plurality of components. In addition, the LSI may be referred to as an IC, a system LSI, a super LSI, or an ultra LSI according to the degree of integration. A computer program for achieving the same operation as each of the above devices is stored in the RAM. The microprocessor operates according to the computer program, so that the functions of the system LSI are achieved. For example, the present invention includes the case where the beamforming method of the present invention is stored as a program of an LSI, the LSI is inserted in a computer, and a predetermined program (beamforming method) is executed.

In addition, the method of IC integration is not limited to an LSI and may be implemented by a dedicated circuit or a general-purpose processor. After LSI fabrication, a field programmable gate array (FPGA) or a reconfigurable processor capable of reconfiguring connection and setting of circuit cells inside the LSI may be used.

Furthermore, if an integrated circuit technology replacing the LSI appears due to advances in semiconductor technology or other derivative technologies, of course, integration of functional blocks may be performed by using the technology.

In addition, some or all of the functions of the ultrasonic diagnostic apparatus according to each embodiment may be implemented by a processor such as a CPU executing a program. A non-transitory computer-readable recording medium in which a program for causing a diagnostic method of the ultrasonic diagnostic apparatus or a beamforming method to be executed is recorded may be employed. A program and signals may be recorded on a recording medium and transported, so that the program may be executed by another independent computer system, or the program may be distributed through a transmission medium such as the Internet.

In the ultrasonic diagnostic apparatus according to the above embodiment, a data storage unit as a storage device is included in the ultrasonic diagnostic apparatus, but the storage device is not limited thereto, and a semiconductor memory, a hard disk drive, an optical disk drive, a magnetic storage device, or the like may be externally connected to the ultrasonic diagnostic apparatus.

The division of functional blocks in the block diagram is merely an example, and a plurality of functional blocks may be implemented as one functional block, one functional block may be divided into a plurality of functional blocks, some functions may be transferred to other functional blocks. In addition, the functions of a plurality of functional blocks having similar functions may be processed by single hardware or software in parallel or in a time division manner.

In addition, the order in which the above steps are executed is exemplified for the purposes of specifically explain the present invention, and an order other than those described above may be used. In addition, a portion of the above steps may be executed simultaneously (in parallel) with other steps.

In addition, the probe and the display unit are externally connected to the ultrasonic diagnostic apparatus, but the probe and the display unit may be integrally provided in the ultrasonic diagnostic apparatus.

In addition, in the above embodiment, the probe illustrates a probe configuration in which a plurality of piezoelectric elements is arranged in a one-dimensional direction. However, the configuration of the probe is not limited thereto. For example, a swing-type probe may be used to obtain a three-dimensional tomographic image by mechanically swinging a two-dimensionally arranged transducer in which a plurality of piezoelectric elements is arranged in a two-dimensional direction or a plurality of transducers arranged in one-dimensional direction or may be used appropriately according to the measurement. For example, in the case of using two-dimensionally arranged probes, the irradiation position and direction of the ultrasonic beam to be transmitted can be controlled by individually changing the timing of applying a voltage to the piezoelectric element and the value of the voltage.

In addition, the probe may include a portion of the functions of the transmission/reception unit in the probe. For example, the transmission electric signal is generated in the probe based on the control signal for generating the transmission electric signal output from the transmission/reception unit, and the transmission electric signal is converted into the ultrasonic wave. In addition, it is possible to adopt a configuration that converts the received reflected ultrasonic wave into the received electric signal and generates the reception signal based on the received electric signal in the probe.

In addition, at least a portion of the functions of the ultrasonic diagnostic apparatus according to each embodiment and modified examples may be combined. Furthermore, all the numbers used above are examples for specifically explaining the present invention, and the present invention is not limited to the exemplified numbers.

In addition, the present invention includes various modified examples where modification is made within the scope conceived by those skilled in the art.

<Summary>

(1) According to an aspect of the present invention, there is provided an ultrasonic diagnostic apparatus that repeats several times a transmission event of transmitting a focused ultrasonic beam to a subject by using a probe having a plurality of transducers, generates a sequence of reception signals by receiving a reflected ultrasonic wave from the subject, and generates an ultrasonic image based on the sequence of reception signals, the ultrasonic diagnostic apparatus including an ultrasonic signal processing circuit including: a transmitter that selects a transmission transducer column from the plurality of transducers for each transmission event while changing a focal point defining a converging position of the ultrasonic beam for each transmission event and transmits the ultrasonic beam from the transmission transducer column to a target region in the subject; a receiver that generates the sequence of reception signals for each transducer based on the reflected ultrasonic wave received from the target region by the probe; a first phasing adder that generates a first acoustic beam signal by performing phasing addition on the sequence of reception signals for a plurality of observation points in a first target region including a portion of the target region for each transmission event; a parameter calculator that calculates a parameter for generating a subframe acoustic beam signal based on the first acoustic beam signal; a second phasing adder that generates a subframe acoustic beam signal by performing phasing addition on the sequence of reception signals based on the parameter for a plurality of observation points in a second target region which is all or a portion of the target region; a synthesizer that generates a frame acoustic beam signal by synthesizing the subframe acoustic beam signals; a controller that determines whether to generate the ultrasonic image based on any one of the first acoustic beam signal and the frame acoustic beam signal; and an ultrasonic image generator that generates the ultrasonic image from any one of the first acoustic beam signal and the frame acoustic beam signal based on the determination of the controller.

With the above-described configuration, it is possible to switch between the operation using the second phasing adder and the operation without using the second phasing adder according to the necessary calculation ability.

(2) In addition, in the ultrasonic diagnostic apparatus according to (1), the ultrasonic signal processing circuit may include a first circuit including the first phasing adder, the parameter calculator, and the controller, and a second circuit including the second phasing adder and the synthesizer, the second circuit may be detachable from the first circuit, and when the second circuit is connected to the first circuit, the controller may determine to generate an ultrasonic image based on the frame acoustic beam signal, and when the second circuit is disconnected from the first circuit, the controller may determine to generate an ultrasonic image based on the first acoustic beam signal.

With the above-described configuration, in the case where emphasis is placed on portability rather than calculation ability, it is possible to generate an ultrasonic image by reception beamforming with a small computation amount, and in the case where emphasis is placed on calculation ability rather than portability, it is possible to generate a high quality ultrasonic image. Therefore, it is possible to perform optimum control according to the usage situation.

(3) In addition, in the ultrasonic diagnostic apparatus according to (1) or (2), the parameter calculator may detect a movement in the subject based on the first acoustic beam signal, and the controller may instruct the second phasing adder to decrease the width of the second target region in a direction in which the transducers of the probe are aligned as the movement in the subject increases.

(4) In addition, in the ultrasonic diagnostic apparatus according to (3), the parameter calculator may detect the movement in the subject from the frame acoustic beam signal generated from the first acoustic beam signal and a frame acoustic beam signal generated from a first acoustic beam signal in a different frame.

With the above-described configuration, it is possible to detect the movement of the probe or the subject without affecting the operation of the second phasing adder, and it is possible to promptly suppress the occurrence of the motion artifact.

(5) In addition, in the ultrasonic diagnostic apparatus according to any one of (1) to (4), the first phasing adder may retain a plurality of estimated values of an ultrasonic velocity in the subject used for the phasing addition and generates a plurality of first acoustic beam signals by using the respective estimated values with respect to the same first target region for each transmission event, the parameter calculator may estimate the ultrasonic velocity in the subject based on the plurality of first acoustic beam signals, and the controller may instruct the second phasing adder to perform the phasing addition using the ultrasonic velocity in the subject estimated by the parameter calculator.

(6) In addition, in the ultrasonic diagnostic apparatus according to (5), the parameter calculator may calculate a turbulence of signal values for each of the plurality of first acoustic beam signals having the same first target region and having different estimated values of an ultrasonic velocity, and the estimated value corresponding to the first acoustic beam signal having the largest turbulence calculated may be estimated to be the ultrasonic velocity in the subject.

With the above-described configurations, it is possible to optimize the ultrasonic velocity and improve the resolution of the ultrasonic image without affecting the calculation of the second phasing adder.

(7) In addition, in the ultrasonic diagnostic apparatus according to any one of (1) to (6), the parameter calculator may calculate a relationship between a depth of the observation point and an S/N ratio of the signal in the first acoustic beam signal and determines an amplification factor according to the depth of the observation point, and the controller may instruct the second phasing adder to perform weighting of a second acoustic beam signal by using the amplification factor according to the depth of the observation point determined by the parameter calculator.

(8) In addition, in the ultrasonic diagnostic apparatus according to (7), the first phasing adder retains a plurality of profiles for a signal amplification factor according to a depth of an observation point and generates a plurality of first acoustic beam signals by using the respective profiles with respect to the same first target region for each transmission event, and the parameter calculator may determine a profile having the smallest turbulence of the S/N ratio of the signal with respect to the depth of the observation point in the first acoustic beam signal as the amplification factor according to the depth of the observation point.

With the above-described configuration, it is possible to control the gain without affecting the operation of the second phasing adder, and it is possible to equalize the S/N ratio of the ultrasonic image.

(9) In addition, in the ultrasonic diagnostic apparatus according to any one of (1) to (8), with respect to a transmission time when the transmitted ultrasonic wave reaches each observation point, in a case where a depth of the observation point is equal to or larger than a focal depth at which the ultrasonic wave is focused in the subject, the second phasing adder may calculate a sum of a first time when the transmitted ultrasonic wave reaches the focal point from the transmission transducer column and a second time when the transmitted ultrasonic wave reaches the observation point from a reference point as the transmission time, and in a case where the depth of the observation point is smaller than the focal depth at which the ultrasonic wave is focused in the subject, the second phasing adder may calculate a result obtained by subtracting the second time from the first time as the transmission time.

With the above-described configuration, in the case of using the second phasing adder, it is possible to generate a high-resolution ultrasonic image.

(10) In addition, in the ultrasonic diagnostic apparatus according to any one of (1) to (9), the ultrasonic image generator may include a first acoustic beam signal synthesizer that generates a frame acoustic beam signal from the first acoustic beam signal, the first target region may be a straight line region which passes through the focal point and is entirely included in the target region, the first phasing adder may generate the first acoustic beam signal by performing the phasing addition including a delay process based on values obtained by dividing, by an ultrasonic velocity in the subject, a distance between the observation point and the focal point and a distance between the focal point and the transducer with respect to the sequence of reception signals corresponding to each transducer included in the transmission transducer column, and, in the case of generating the ultrasonic image from the first acoustic beam signal, the ultrasonic image generator may generate the ultrasonic image from the frame acoustic beam signal generated by the first acoustic beam signal synthesizer.

(11) In addition, in the ultrasonic diagnostic apparatus according to (10), with respect to a transmission time when the transmitted ultrasonic wave reaches each observation point, in a case where a depth of the observation point is equal to or larger than a focal depth at which the ultrasonic wave is focused in the subject, the first phasing adder may calculate a sum of a first time when the transmitted ultrasonic wave reaches the focal point from the transmission transducer column and a second time when the transmitted ultrasonic wave reaches the observation point from a reference point as the transmission time, and in a case where the depth of the observation point is smaller than the focal depth at which the ultrasonic wave is focused in the subject, the first phasing adder may calculate a result obtained by subtracting the second time from the first time as the transmission time.

With the above-described configuration, in the case of not using the first phasing adder, it is possible to generate an ultrasonic image with reduced quality deterioration while greatly reducing the calculation amount. In addition, in the case of using the second phasing adder, it is possible to reduce the error particularly when calculating the ultrasonic velocity as a parameter.

(12) In addition, in the ultrasonic diagnostic apparatus according to (10) or (11), with respect to a reception time when a reflected wave from each observation point reaches each transducer, the first phasing adder may calculate a time until the reflected wave from the observation point reaches the transducer closest to the observation point as the reception time corresponding to the transducer closest to the observation point and calculate, as the reception time corresponding to the transducer, a time obtained by adding a difference between a time until the ultrasonic wave reaches from the focal point to the transducer and a time until the ultrasonic wave reaches from the focal point to the transducer closest to the observation point to the reception time corresponding to the transducer closest to the observation point.

With the above-described configuration, it becomes unnecessary to identify the reception signal for each observation point, and it is possible to greatly reduce the calculation amount of the first phasing adder.

(13) In addition, in the ultrasonic diagnostic apparatus according to any one of (10) to (12), for each transmission event with respect to each observation point in the target region, the first acoustic beam signal synthesizer may generate the subframe acoustic beam signals by allocating the first acoustic beam signal of the observation point of which distance to the focal point is the same as the observation point and which exists on the straight line as the acoustic beam signal of the observation point and generate the frame acoustic beam signal by synthesizing the generated plurality of subframe acoustic beam signals.

With the above-described configuration, it is possible to reduce the calculation amount of the first phasing adder with respect to the number of observation points in the target region.

(14) In addition, in the ultrasonic diagnostic apparatus according to any one of (1) to (9), the first target region may be configured with one or more straight lines which pass through the focal point or the vicinity thereof and are perpendicular to the direction in which the transducers of the probe are aligned, the first phasing adder may perform phasing addition on a value obtained by dividing the depth of the observation point by an ultrasonic velocity in the subject as a transmission time and a value obtained by dividing a distance from the observation point to the transducer by the ultrasonic velocity in the subject as a reception time to generate the first acoustic beam signal, and in the case of generating an ultrasonic image from the first acoustic beam signal, the ultrasonic image generator may generate the ultrasonic image from the plurality of first acoustic beam signals generated by the first phasing adder.

With the above-described configuration, in the case of not using the first phasing adder, it is possible to reduce the calculation amount.

(15) In addition, according to an aspect of the present invention, there is provided an ultrasonic diagnostic apparatus that repeats several times a transmission event of transmitting a focused ultrasonic beam to a subject by using a probe having a plurality of transducers, generates a sequence of reception signals by receiving a reflected ultrasonic wave from the subject, and generates an ultrasonic image based on the sequence of reception signals, the ultrasonic diagnostic apparatus including an ultrasonic signal processing circuit including: a transmitter that selects a transmission transducer column from the plurality of transducers for each transmission event while changing a focal point defining a converging position of the ultrasonic beam for each transmission event and transmits the ultrasonic beam from the transmission transducer column to a target region in the subject; a receiver that generates the sequence of reception signals for each transducer based on the reflected ultrasonic wave received from the target region by the probe; a first phasing adder that generates a first acoustic beam signal by performing phasing addition on the sequence of reception signals for a plurality of observation points in a first target region including a portion of the target region for each transmission event; a parameter calculator that calculates a parameter for generating a subframe acoustic beam signal based on the first acoustic beam signal; an ultrasonic image generator that generates an ultrasonic image from the first acoustic beam signal; and a controller, wherein an arithmetic circuit is detachable from the ultrasonic signal processing circuit, the arithmetic circuit including: a second phasing adder that generates a subframe acoustic beam signal by performing phasing addition on the sequence of reception signals based on the parameter for a plurality of observation points in a second target region which is all or a portion of the target region; and a synthesizer that generates a frame acoustic beam signal by synthesizing the subframe acoustic beam signals, and when the arithmetic circuit is connected to the ultrasonic signal processing circuit, the controller instructs the ultrasonic image generator to generate the ultrasonic image from the frame acoustic beam signal instead of the first acoustic beam signal.

With the above-described configuration, it is possible to generate an ultrasonic image by reception beamforming with a small calculation amount based on the first acoustic beam signal generated by the first phasing adder. On the other hand, in the case where a high calculation ability is required, high quality ultrasonic images can be generated by attaching the second phasing adder and the synthesizer. Furthermore, by performing the parameter control on the second phasing adder based on the first acoustic beam signal, it is possible to optimize the parameter by the processing of the low calculation amount by the first phasing adder without unnecessarily repeating the reception beamforming by the second phasing adder with a large calculation amount.

The ultrasonic diagnostic apparatus according to the present disclosure can be used both as a portable ultrasonic diagnostic apparatus and as an ultrasonic diagnostic apparatus with high calculation ability and can perform optimum operation according to the usage situation.

Although embodiments of the present invention have been described and illustrated in detail, the disclosed embodiments are made for purposes of illustration and example only and not limitation. The scope of the present invention should be interpreted by terms of the appended claims.

**Claims**

1. An ultrasonic diagnostic apparatus (100) that repeats several times a transmission event of transmitting a focused ultrasonic beam to a subject by using a probe (101, 201) having a plurality of transducers (101a), generates a sequence of reception signals by receiving a reflected ultrasonic wave from the subject, and generates an ultrasonic image based on the sequence of reception signals, the ultrasonic diagnostic apparatus (100) comprising an ultrasonic signal processing circuit including:

   a transmitter (1031) that selects a transmission transducer (101a) column from the plurality of transducers (101a) for each transmission event while changing a focal point defining a converging position of the ultrasonic beam for each transmission event and transmits the ultrasonic beam from the transmission transducer (101a) column to a target region in the subject;
   a receiver (1040) that generates the sequence of reception signals for each transducer (101a) based on the reflected ultrasonic wave received from the target region by the probe (101, 201);
   a first phasing adder (1041) that generates a first acoustic beam signal by performing phasing addition on the sequence of reception signals for a plurality of observation points in a first target region including a portion of the target region for each transmission event;
   a parameter calculator (1043) that calculates a parameter for generating a subframe acoustic beam signal based on the first acoustic beam signal;
   a second phasing adder (1042) that generates a subframe acoustic beam signal by performing phasing addition on the sequence of reception signals based on the parameter for a plurality of observation points in a second target region which is all or a portion of the target region;
   a synthesizer (1044, 1045) that generates a frame acoustic beam signal by synthesizing the subframe acoustic beam signals;
   a controller (108) that determines whether to generate the ultrasonic image based on any one of the first acoustic beam signal and the frame acoustic beam signal; and
   an ultrasonic image generator (105) that generates the ultrasonic image from any one of the first acoustic beam signal and the frame acoustic beam signal based on the determination of the controller (108).

2. The ultrasonic diagnostic apparatus (100) according to claim 1,

   wherein the ultrasonic signal processing circuit includes a first circuit including the first phasing adder (1041), the parameter calculator (1043), and the controller (108), and a second circuit including the second phasing adder (1042) and the synthesizer (1044, 1045),
   the second circuit is detachable from the first circuit, and
   when the second circuit is connected to the first circuit, the controller (108) determines to generate an ultrasonic image based on the frame acoustic beam signal, and when the second circuit is disconnected from the first circuit, the controller (108) determines to generate an ultrasonic image based on the first acoustic beam signal.

3. The ultrasonic diagnostic apparatus (100) according to claim 1 or 2,

wherein the parameter calculator (1043) detects a movement in the subject based on the first acoustic beam signal, and the controller (108) instructs the second phasing adder (1042) to decrease the width of the second target region in a direction in which the transducers (101a) of the probe (101, 201) are aligned as the movement in the subject increases.

4. The ultrasonic diagnostic apparatus (100) according to claim 3, wherein the parameter calculator (1043) detects the movement in the subject from the frame acoustic beam signal generated from the first acoustic beam signal and a frame acoustic beam signal generated from a first acoustic beam signal in a different frame.

5. The ultrasonic diagnostic apparatus (100) according to any one of claims 1 to 4,

wherein the first phasing adder (1041) retains a plurality of estimated values of an ultrasonic velocity in the subject used for the phasing addition and generates a plurality of first acoustic beam signals by using the respective estimated values with respect to the same first target region for each transmission event, the parameter calculator (1043) estimates the ultrasonic velocity in the subject based on the plurality of first acoustic beam signals, and the controller (108) instructs the second phasing adder (1042) to perform the phasing addition using the ultrasonic velocity in the subject estimated by the parameter calculator (1043).

6. The ultrasonic diagnostic apparatus (100) according to claim 5,

wherein the parameter calculator (1043) calculates a turbulence of signal values for each of the plurality of first acoustic beam signals having the same first target region and having different estimated values of an ultrasonic velocity, and the estimated value corresponding to the first acoustic beam signal having the largest turbulence calculated is estimated to be the ultrasonic velocity in the subject.

7. The ultrasonic diagnostic apparatus (100) according to any one of claims 1 to 6,

wherein the parameter calculator (1043) calculates a relationship between a depth of the observation point and an S/N ratio of the signal in the first acoustic beam signal and determines an amplification factor according to the depth of the observation point, and the controller (108) instructs the second phasing adder (1042) to perform weighting of a second acoustic beam signal by using the amplification factor according to the depth of the observation point determined by the parameter calculator (1043).

8. The ultrasonic diagnostic apparatus (100) according to claim 7,

wherein the first phasing adder (1041) retains a plurality of profiles for a signal amplification factor according to a depth of an observation point and generates a plurality of first acoustic beam signals by using the respective profiles with respect to the same first target region for each transmission event, and the parameter calculator (1043) determines a profile having the smallest turbulence of the S/N ratio of the signal with respect to the depth of the observation point in the first acoustic beam signal as the amplification factor according to the depth of the observation point.

9. The ultrasonic diagnostic apparatus (100) according to any one of claims 1 to 8, wherein, with respect to a transmission time when the transmitted ultrasonic wave reaches each observation point, in a case where a depth of the observation point is equal to or larger than a focal depth at which the ultrasonic wave is focused in the subject, the second phasing adder (1042) calculates a sum of a first time when the transmitted ultrasonic wave reaches the focal point from the transmission transducer (101a) column and a second time when the transmitted ultrasonic wave reaches the observation point from a reference point as the transmission time, and in a case where the depth of the observation point is smaller than the focal depth at which the ultrasonic wave is focused in the subject, the second phasing adder (1042) calculates a result obtained by subtracting the second time from the first time as the transmission time.

10. The ultrasonic diagnostic apparatus (100) according to any one of claims 1 to 9,

wherein the ultrasonic image generator (105) includes a first acoustic beam signal synthesizer (1044, 1045) that generates a frame acoustic beam signal from the first acoustic beam signal, the first target region is a straight line region which passes through the focal point and is entirely included in the target region, the first phasing adder (1041) generates the first acoustic beam signal by performing the phasing addition including a delay process based on values obtained by dividing, by an ultrasonic velocity in the subject, a distance between the observation point and the focal point and a distance between the focal point and the transducer (101a) with respect to the sequence of reception signals corresponding to each transducer (101a) included in the transmission transducer (101a) column, and

in the case of generating the ultrasonic image from the first acoustic beam signal, the ultrasonic image generator (105) generates the ultrasonic image from the frame acoustic beam signal generated by the first acoustic beam signal synthesizer (1044, 1045).

11. The ultrasonic diagnostic apparatus (100) according to claim 10, wherein, with respect to a transmission time when the transmitted ultrasonic wave reaches each observation point, in a case where a depth of the observation point is equal to or larger than a focal depth at which the ultrasonic wave is focused in the subject, the first phasing adder (1041) calculates a sum of a first time when the transmitted ultrasonic wave reaches the focal point from the transmission transducer (101a) column and a second time when the transmitted ultrasonic wave reaches the observation point from a reference point as the transmission time, and in a case where the depth of the observation point is smaller than the focal depth at which the ultrasonic wave is focused in the subject, the first phasing adder (1041) calculates a result obtained by subtracting the second time from the first time as the transmission time.

12. The ultrasonic diagnostic apparatus (100) according to claim 10 or 11, wherein, with respect to a reception time when a reflected wave from each observation point reaches each transducer (101a), the first phasing adder (1041) calculates a time until the reflected wave from the observation point reaches the transducer (101a) closest to the observation point as the reception time corresponding to the transducer (101a) closest to the observation point and calculates, as the reception time corresponding to the transducer (101a), a time obtained by adding a difference between a time until the ultrasonic wave reaches from the focal point to the transducer (101a) and a time until the ultrasonic wave reaches from the focal point to the transducer (101a) closest to the observation point to the reception time corresponding to the transducer (101a) closest to the observation point.

13. The ultrasonic diagnostic apparatus (100) according to any one of claims 10 to 12, wherein, for each transmission event with respect to each observation point in the target region, the first acoustic beam signal synthesizer (1044, 1045) generates the subframe acoustic beam signals by allocating the first acoustic beam signal of the observation point of which distance to the focal point is the same as the observation point and which exists on the straight line as the acoustic beam signal of the observation point and generates the frame acoustic beam signal by synthesizing the generated plurality of subframe acoustic beam signals.

14. The ultrasonic diagnostic apparatus (100) according to any one of claims 1 to 9,

wherein the first target region is configured with one or more straight lines which pass through the focal point or the vicinity thereof and are perpendicular to the direction in which the transducers (101a) of the probe (101, 201) are aligned, the first phasing adder (1041) performs phasing addition on a value obtained by dividing the depth of the observation point by an ultrasonic velocity in the subject as a transmission time and a value obtained by dividing a distance from the observation point to the transducer (101a) by the ultrasonic velocity in the subject as a reception time to generate the first acoustic beam signal, and

in the case of generating an ultrasonic image from the first acoustic beam signal, the ultrasonic image generator (105) generates the ultrasonic image from the plurality of first acoustic beam signals generated by the first phasing adder (1041).

15. An ultrasonic diagnostic apparatus (100) that repeats several times a transmission event of transmitting a focused ultrasonic beam to a subject by using a probe (101, 201) having a plurality of transducers (101a), generates a sequence of reception signals by receiving a reflected ultrasonic wave from the subject, and generates an ultrasonic image based on the sequence of reception signals, the ultrasonic diagnostic apparatus (100) comprising an ultrasonic signal processing circuit including:

a transmitter (1031) that selects a transmission transducer (101a) column from the plurality of transducers (101a) for each transmission event while changing a focal point defining a converging position of the ultrasonic beam for each transmission event and transmits the ultrasonic beam from the transmission transducer (101a) column to a target region in the subject; a receiver (1040) that generates the sequence of reception signals for each transducer (101a) based on the reflected ultrasonic wave received from the target region by the probe (101, 201); a first phasing adder (1041) that generates a first acoustic beam signal by performing phasing addition on the sequence of reception signals for a plurality of observation points in a first target region including a portion of the target region for each transmission event;

a parameter calculator (1043) that calculates a parameter for generating a subframe acoustic beam signal based on the first acoustic beam signal;

an ultrasonic image generator (105) that generates an ultrasonic image from the first acoustic beam signal; and

a controller (108),

wherein an arithmetic circuit (1047) is detachable from the ultrasonic signal processing circuit, the arithmetic circuit (1047) including:

a second phasing adder (1042) that generates a subframe acoustic beam signal by performing phasing addition on the sequence of reception signals based on the parameter for a plurality of observation points in a second target region which is all or a portion of the target region; and

a synthesizer (1044, 1045) that generates a frame acoustic beam signal by synthesizing the subframe acoustic beam signals, and

when the arithmetic circuit (1047) is connected to the ultrasonic signal processing circuit, the controller (108) instructs the ultrasonic image generator (105) to generate the ultrasonic image from the frame acoustic beam signal instead of the first acoustic beam signal.

*FIG. 1*

ULTRASONIC DIAGNOSTIC APPARATUS — 1000

PROBE — 101
101a

ULTRASONIC SIGNAL PROCESSING APPARATUS — 150 / 100

MULTIPLEXER UNIT — 102

TRANSMISSION BEAMFORMER UNIT — 103
TRANSMITTER — 1031

RECEPTION BEAMFORMER UNIT — 104

ULTRASONIC IMAGE GENERATOR — 105

DISPLAY UNIT — 106

CONTROLLER — 108

DATA STORAGE UNIT — 107

# FIG. 2

ULTRASONIC PRIMARY
IRRADIATION REGION Ax

TRANSMISSION FOCAL POINT F

TRANSMISSION FOCAL DEPTH

101a

Y
X

F

TRANSMISSION
APERTURE Tx

MOVE BY MOVING PITCH Mp
FOR EACH TRANSMISSION EVENT
(ELECTRON SCAN)

## FIG. 3

FIG. 3

104 — RECEPTION BEAMFORMER UNIT

103    108

1041 — FIRST PHASING ADDER

1044 — FIRST SYNTHESIZER

1046

1040 — RECEIVER

102

1043 — PARAMETER CALCULATOR

105

108

1047

1042 — SECOND PHASING ADDER

1045 — SECOND SYNTHESIZER

107

SEQUENCE OF RECEPTION SIGNALS

SUBFRAME ACOUSTIC BEAM SIGNAL

BEAM-REGION ACOUSTIC BEAM SIGNAL

SECOND FRAME ACOUSTIC BEAM SIGNAL

FIRST FRAME ACOUSTIC BEAM SIGNAL

EP 3 349 034 A2

FIG. 4

# FIG. 5

OBSERVATION LINE BL
(SETTING REGION OF
REPRESENTATIVE POINT Qk)

REPRESENTATIVE POINT Q

Qk

TARGET REGION Bx

ULTRASONIC PRIMARY
IRRADIATION REGION Ax

F — TRANSMISSION FOCAL POINT F

Y
X

101a

## FIG. 6A

REPRESENTATIVE POINT Q

OBSERVATION LINE BL

Qk

ULTRASONIC PRIMARY IRRADIATION REGION Ax

402

TRANSMISSION FOCAL POINT F

F

403

Y

401

101a

X

TRANSMISSION APERTURE Tx

RECEPTION TRANSDUCER Rm

## FIG. 6B

OBSERVATION LINE BL

ULTRASONIC PRIMARY IRRADIATION REGION Ax

TRANSMISSION FOCAL POINT F

405    F

REPRESENTATIVE POINT Q

Qk

Y

401

406

404

403

X

101a

TRANSMISSION APERTURE Tx

RECEPTION TRANSDUCER Rm

# FIG. 7

TRANSMISSION FOCAL POINT F

TRANSMISSION APERTURE Tx

# FIG. 8

EP 3 349 034 A2

# FIG. 9

## FIG. 10A

TARGET REGION Cx

OBSERVATION POINT P

Pij

402

ULTRASONIC PRIMARY IRRADIATION REGION Ax

TRANSMISSION FOCAL POINT F

403

F

Y

X

401

101a

RECEPTION TRANSDUCER Rij

TRANSMISSION APERTURE Tx

## FIG. 10B

TARGET REGION Cx

ULTRASONIC PRIMARY IRRADIATION REGION Ax

TRANSMISSION FOCAL POINT F

402  F

OBSERVATION POINT P

Pij

Y

403

401

404

X

101a

RECEPTION TRANSDUCER Rij

TRANSMISSION APERTURE Tx

## FIG. 11A

## FIG. 11B

## FIG. 12A

1044

FIRST SYNTHESIZER

1341
ACOUSTIC BEAM SIGNAL
DEVELOPMENT UNIT

1342
WEIGHTING
SYNTHESIZER

1046

107

BEAM-REGION
ACOUSTIC BEAM SIGNAL

FIRST FRAME
ACOUSTIC BEAM SIGNAL

## FIG. 12B

1045

SECOND SYNTHESIZER

1043
ADDITION
PROCESSING UNIT

1344
AMPLIFICATION
PROCESSING UNIT

1046

107

SUBFRAME ACOUSTIC
BEAM SIGNAL

SECOND FRAME
ACOUSTIC BEAM SIGNAL

# FIG. 13

# FIG. 14

TARGET REGION Bx

OBSERVATION
POINT S

TRANSMISSION
FOCAL POINT F

F

Y

X

101a

TRANSMISSION
APERTURE Tx

Pijo

WEIGHT
DISTRIBUTION

601

EP 3 349 034 A2

## FIG. 15A

## FIG. 15B

DEPTH

DEPTH

$P = Pmax$

MAXIMUM NUMBER
OF TIMES OF
SUPERIMPOSITION

AMPLIFICATION
FACTOR

*FIG. 16*

START

TRANSMISSION PROCESS — S101

RECEPTION PROCESS — S102

S103
HAS TRANSMISSION WITH RESPECT TO ALL TRANSDUCERS BEEN COMPLETED?

NO

YES

SET TARGET REGION Bx — S201

SET TARGET LINE BL (REPRESENTATIVE POINT Qk EXISTING) — S202

GENERATE BEAM-REGION ACOUSTIC BEAM SIGNAL WITH RESPECT TO REPRESENTATIVE POINT Qk — S210

CALCULATE PARAMETER BASED ON BEAM-REGION ACOUSTIC BEAM SIGNAL — S211

GENERATE SUBFRAME ACOUSTIC BEAM SIGNAL WITH RESPECT TO OBSERVATION POINT Pij USING PARAMETER — S220

S230
HAS PROCESS WITH RESPECT TO ALL TRANSMISSION EVENTS ENDED?

NO

YES

GENERATE FRAME ACOUSTIC BEAM SIGNAL BY WEIGHTING AND ADDING SUBFRAME ACOUSTIC BEAM SIGNAL USING POSITION OF OBSERVATION POINT AS INDEX — S301

MULTIPLY EACH OF SYNTHESIZED ACOUSTIC BEAM SIGNALS OF FRAME ACOUSTIC BEAM SIGNAL BY AMPLIFICATION FACTOR DETERMINED ACCORDING TO NUMBER OF TIMES OF ADDITION — S302

GENERATE ULTRASONIC IMAGE BASED ON FRAME ACOUSTIC BEAM SIGNAL — S303

END

EP 3 349 034 A2

# FIG. 17

```
┌──────────────────────────────────────────┐
│  GENERATION OF ACOUSTIC BEAM SIGNAL      │
│  WITH RESPECT TO REPRESENTATIVE POINT Qk │
└──────────────────────────────────────────┘
```

CALCULATE FIRST TIME FOR TRANSMITTED ULTRASONIC — S2111
WAVE TO REACH TRANSMISSION FOCAL POINT F

CALCULATE SECOND TIME FOR ULTRASONIC — S2112
WAVE TO REACH REPRESENTATIVE POINT Qk
FROM TRANSMISSION FOCAL POINT F

m ← INITIAL VALUE — S2113

CALCULATE THIRD TIME FOR ULTRASONIC WAVE TO REACH — S2114
EACH RECEPTION TRANSDUCER Rm IN TRANSMISSION
APERTURE FROM TRANSMISSION FOCAL POINT F

CALCULATE TOTAL PROPAGATION TIME — S2115
TO REACH EACH RECEPTION TRANSDUCER Rm

S2118  CALCULATE DELAY AMOUNT APPLIED TO SEQUENCE OF — S2116
RECEPTION SIGNALS OF EACH RECEPTION TRANSDUCER Rm

m=m+1                                        S2117

NO ◇ HAS PROCESS WITH RESPECT TO ALL m ENDED? ◇

↓ YES

PERFORM DELAY PROCESS ON SEQUENCE OF RECEPTION — S2121
SIGNALS OF EACH RECEPTION TRANSDUCER Rm AND
SYNCHRONIZE TIME POINT OF RECEPTION SIGNAL
CORRESPONDING TO REPRESENTATIVE POINT Qk

CALCULATE WEIGHT SERIES CORRESPONDING TO — S2122
EACH RECEPTION TRANSDUCER Rm
FOR EACH DEPTH OF REPRESENTATIVE POINT Qk

MULTIPLY AND ADD WEIGHT FOR EACH DEPTH AND — S2123
EACH RECEPTION TRANSDUCER Rm
TO SEQUENCE OF SYNCHRONIZED RECEPTION SIGNALS

OUTPUT BEAM-REGION ACOUSTIC BEAM SIGNAL — S2124

( END )

# FIG. 18

# FIG. 19

START OF GENERATION OF SUBFRAME
ACOUSTIC BEAM SIGNAL WITH RESPECT TO Pij

SET TARGET REGION Cx (OBSERVATION POINT Pij EXISTING) — S2210

i ← INITIAL VALUE — S2211

j ← INITIAL VALUE — S2212

SET RECEPTION APERTURE WITH RESPECT TO OBSERVATION
POINT Pij (OBSERVATION POINT SYNCHRONIZATION TYPE) — S2231

CALCULATE TRANSMISSION TIME FOR TRANSMITTED
ULTRASONIC WAVE TO REACH OBSERVATION POINT Pij — S2241

k ← INITIAL VALUE — S2242

CALCULATE RECEPTION TIME FOR REFLECTED WAVE FROM OBSERVATION
POINT Pij TO REACH EACH RECEPTION TRANSDUCER Rk IN RECEPTION APERTURE — S2243

CALCULATE TOTAL PROPAGATION TIME TO REACH EACH RECEPTION TRANSDUCER Rk — S2244

CALCULATE DELAY AMOUNT APPLIED TO SEQUENCE OF
RECEPTION SIGNALS OF EACH RECEPTION TRANSDUCER Rk — S2245

S2264   i ← i+1

S2262   j ← j+1

S2247   k ← k+1

NO   HAS PROCESS WITH RESPECT TO ALL k ENDED? — S2246

↓ YES

IDENTIFY RECEPTION SIGNAL CORRESPONDING TO DELAY AMOUNT FROM
SEQUENCE OF RECEPTION SIGNALS OF EACH RECEPTION TRANSDUCER Rk — S2248

CALCULATE WEIGHT SERIES CORRESPONDING TO EACH RECEPTION TRANSDUCER Rk — S2249

MULTIPLY AND ADD WEIGHT FOR EACH RECEPTION TRANSDUCER Rk TO RECEPTION
SIGNAL IDENTIFIED CORRESPONDING TO EACH RECEPTION TRANSDUCER Rk — S2250

OUTPUT ACOUSTIC BEAM SIGNAL CORRESPONDING
TO OBSERVATION POINT Pij TO DATA STORAGE UNIT — S2251

NO   HAS PROCESS WITH RESPECT TO ALL j ENDED? — S2261

↓ YES   S2263

NO   HAS PROCESS WITH RESPECT TO ALL i ENDED?

↓ YES

RETURN

# FIG. 20

# FIG. 21

START

TRANSMISSION PROCESS — S101

RECEPTION PROCESS — S102

S103
HAS TRANSMISSION
WITH RESPECT TO ALL TRANSDUCERS
BEEN COMPLETED?

NO

YES

SET TARGET REGION Bx
(OBSERVATION POINT Sij EXISTING) — S201

SET TARGET LINE BL
(REPRESENTATIVE POINT Qk EXISTING) — S202

GENERATE BEAM-REGION ACOUSTIC BEAM SIGNAL
WITH RESPECT TO REPRESENTATIVE POINT Qk — S210

GENERATE ACOUSTIC BEAM OF OBSERVATION POINT Sij
FROM ACOUSTIC BEAM OF REPRESENTATIVE POINT Qk
BASED ON DISTANCE TO TRANSMISSION FOCAL POINT F — S212

S230
HAS PROCESS
WITH RESPECT TO ALL TRANSMISSION
EVENTS ENDED?

NO

YES

GENERATE FRAME ACOUSTIC BEAM SIGNAL BY WEIGHTING
AND ADDING SUBFRAME ACOUSTIC BEAM SIGNAL
USING POSITION OF OBSERVATION POINT AS INDEX — S311

MULTIPLY EACH OF SYNTHESIZED ACOUSTIC
BEAM SIGNALS OF FRAME ACOUSTIC BEAM SIGNAL
BY AMPLIFICATION FACTOR DETERMINED
ACCORDING TO NUMBER OF TIMES OF ADDITION — S312

GENERATE ULTRASONIC IMAGE BASED
ON FRAME ACOUSTIC BEAM SIGNAL — S313

END

# FIG. 22

# FIG. 23

FIG. 24A

FIG. 24B

FIG. 24C

EP 3 349 034 A2

## FIG. 25

*RECEPTION BEAMFORMER UNIT* (304)

RECEIVER (1040)

FIRST PHASING ADDER (1041) — 103, 108

FIRST SYNTHESIZER (1044) — 108

PARAMETER CALCULATOR (1043)

1046 — 105

SEQUENCE OF RECEPTION SIGNALS

BEAM-REGION ACOUSTIC BEAM SIGNAL

FIRST FRAME ACOUSTIC BEAM SIGNAL (107)

102

EP 3 349 034 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006113445 A

**Non-patent literature cited in the description**

- **MASAYASU ITO ; TSUYOSHI MOCHIZUKI.** Ultrasonic Diagnostic Apparatus. Corona Publishing Co., Ltd, 26 August 2002, 42, , 45
- **S.I. NIKOLOV ; J.A. JENSEN.** Virtual ultrasound sources in high resolution ultrasound imaging. *Proc, SPIE - Progress in biomedical optics and imaging,* 2002, vol. 3, 395-405
- **JACOB KORTBEK.** Synthetic Aperture Sequential Beamforming. *IEEE Ultrasonics Symposium,* 02 November 2008, 966-969